(19) Europäisches Patentamt
European Patent Office
Office européen des brevets

(11) EP 0 587 193 B1

(12) EUROPEAN PATENT SPECIFICATION

(45) Date of publication and mention of the grant of the patent:
**02.01.1997 Bulletin 1997/01**

(21) Application number: **93115717.6**

(22) Date of filing: **13.02.1990**

(51) Int Cl.6: **C07C 217/10**, A61K 31/13, A61K 31/33, A61K 31/21, C07D 295/088, C07D 207/27, C07C 323/19, C07D 317/54, C07D 211/20, C07D 333/16, C07D 207/08

(54) **1,2-Ethanediol derivative and salt thereof, process for producing the same, and cerebral function-improving agent comprising the same**

1,2-Ethandiolderivate und ihre Salze, Verfahren zu ihrer Herstellung und sie enthaltende gehirnfunktionsverbessernde Mittel

Dérivés d'éthanediol-1,2 et leurs sels, procédé de leur préparation et agents rétablissant la fonction cérébrale les contenant

(84) Designated Contracting States:
**AT CH DE DK GB LI NL SE**

(30) Priority: **14.02.1989 JP 32714/89**
**20.03.1989 JP 68958/89**
**26.04.1989 JP 106187/89**
**05.02.1990 JP 24501/90**
**05.02.1990 JP 24502/90**
**05.02.1990 JP 24503/90**

(43) Date of publication of application:
**16.03.1994 Bulletin 1994/11**

(62) Application number of earlier application in accordance with Art. 76 EPC: **90102829.0**

(73) Proprietor: **TOYAMA CHEMICAL CO., LTD.**
**Tokyo 160 (JP)**

(72) Inventors:
- **Ono, Satoshi**
  **Toyama-shi (JP)**
- **Yamafuji, Tetsuo**
  **Nei-gun, Toyama-ken (JP)**
- **Chaki, Hisaaki**
  **Kaminiikawa-gun, Toyama-ken (JP)**
- **Maekawa, Mutsuko**
  **Toyama-shi (JP)**
- **Todo, Yozo**
  **Toyama-shi (JP)**
- **Narita, Hirokazu**
  **Toyama-shi (JP)**

(74) Representative:
**Wächtershäuser, Günter, Prof. Dr.**
**Patentanwalt,**
**Tal 29**
**80331 München (DE)**

(56) References cited:
**WO-A-88/08424** **US-A- 2 928 845**

- **CHEMICAL ABSTRACTS, vol. 63, no. 1, 5th July 1965, column 557, abstract no. 557h, Columbus, Ohio, US; B. MACCHIA: "Stereochemistry and orientation of the addition of aminoalcohols to oxides of cis- and trans-propenylbenzene"**
- **JOURNAL OF PHARMACEUTICAL SCIENCES, vol. 50, 1961, pages 769-771; S.L. SHAPIRO et al.: "Local anesthetics III. Dialkylamino-alkoxyphenylethanol esters"**
- **IL FARMACO EDIZIONE SCIENTIFICA, vol. 19, 1964, pages 1056-1065; F. BOTTARI et al.: "Eteri del fenilglicol e di idrobenzoine con amminoalcoli"**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

This invention relates to a 1,2-ethanediol derivative and a salt thereof, a process for producing the same, and a cerebral function-improving agent comprising the same. The cerebral function-improving agent of this invention is useful for treating cerebrovascular dementia, senile dementia, Alzheimer's dementia, sequelae of ischemic encephalopathy and cerebral apoplexy.

As 1,2-ethanediol derivatives, there are known, for example, those described in U.S. Patent No. 2,928,845; J. Pharm. Sci., vol. 50, pp. 769-771 (1961); Farmaco. Ed. Sci., vol. 19, pp. 1056-1065 (1964); etc.

These compounds are in use as a local anesthetic. However, nothing is known as to their use as a cerebral function-improving agent, an antiamnesic agent or a nootropic agent.

WO No. 88/8424 describes that 1,2-ethanediol derivatives can be used for the remedy of Alzheimer's disease and other degenerative neurological disorders. However, no specific description or example of these derivatives is given in the application.

Drugs such as cerebral metabolic enhancers, cerebrovasodilators and the like are currently in use for the remedy of various dementias, particularly dementia of Alzheimer's type and cerebrovascular dementia.

However, no cerebral function-improving agent has been found as yet which is useful for treating cerebrovascular dementia, senile dementia, Alzheimer's dementia, sequelae of ischemic encephalopathy and cerebral apoplexy.

An object of this invention is to provide a novel 1,2-ethanediol derivative and a salt thereof.

Another object of this invention is to provide a process for producing a novel 1,2-ethanediol derivative and a salt thereof.

Still another object of this invention is to provide a novel cerebral function-improving agent which is useful for treating cerebrovascular dementia, senile dementia, Alzheimer's dementia, sequelae of ischemic encephalopathy and cerebral apoplexy and yet has little side effect.

The present inventors have made study in order to solve the above-mentioned problems. As a result, it has been found that a 1,2-ethanediol derivative represented by the following general formula [I] or a salt thereof has an excellent antiamnesic activity and an excellent antihypoxic activity and is very useful as a cerebral function-improving agent:

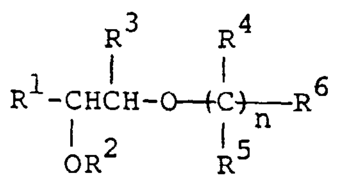

[I]

wherein $R^1$ represents a substituted or unsubstituted phenyl group; $R^2$ represents a hydrogen atom, a $C_1$-$C_6$ alkyl group or a hydroxyl-protecting group; $R^3$ represents a hydrogen atom or a $C_1$-$C_6$ alkyl group; $nR^4$'s and $nR^5$'s are the same as or different from one another and represent hydrogen atoms or $C_1$-$C_6$ alkyl groups; $R^6$ represents an ammonio group or a substituted or unsubstituted amino or nitrogen-containing heterocyclic group, the nitrogen-containing heterocyclic group having a free valence on a nitrogen atom forming the heterocyclic ring and being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6; wherein the substituent on $R^1$ is selected from the group consisting of halogen atoms, substituted or unsubstituted amino, $C_1$-$C_6$ alkyl, aryl, ar-$C_1$-$C_4$ alkyl, $C_1$-$C_6$ alkoxy, ar-$C_1$-$C_4$ alkoxy, aryloxy, carbamoyloxy, $C_1$-$C_6$ alkylthio, $C_2$-$C_6$ alkenyl, $C_2$-$C_6$ alkenyloxy, ar-$C_1$-$C_4$ alkylthio, ar-$C_1$-$C_6$ alkylsulfonyl, arylsulfonyl, $C_1$-$C_6$ alkylsulfonylamino, arylsulfonylamino and heterocyclic groups and protected amino groups, protected or unprotected hydroxyl groups, nitro group, oxo group and $C_1$-$C_4$ alkylenedioxy groups; the substituted $C_1$-$C_6$ alkyl, aryl, ar-$C_1$-$C_4$ alkyl, $C_1$-$C_6$ alkoxy, ar-$C_1$-$C_4$ alkoxy, aryloxy, carbamoyloxy, $C_1$-$C_6$ alkylthio, $C_2$-$C_6$ alkenyl, $C_2$-$C_6$ alkenyloxy, ar-$C_1$-$C_4$ alkylthio, ar-$C_1$-$C_6$ alkylsulfonyl, arylsulfonyl, $C_1$-$C_6$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected hydroxyl groups, protected or unprotected amino groups, unsubstituted $C_1$-$C_6$ alkyl groups, $C_1$-$C_6$ alkyl groups substituted by a protected or unprotected hydroxyl group, halogen-substituted aryl groups, halogen-substituted aroyl groups, unsubstituted $C_1$-$C_6$ alkoxy groups, $C_1$-$C_6$ alkoxy groups substituted by a $C_1$-$C_6$ alkoxy group, $C_1$-$C_6$ acyl groups, ar-$C_1$-$C_4$ alkenyl groups, heterocyclic groups, oxo group, $C_1$-$C_6$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_1$-$C_6$ alkyl groups, $C_1$-$C_6$ alkyl groups substi-

tuted by a protected or unprotected carboxyl or hydroxyl group, cycloalkyl groups, aryl groups, $C_1$-$C_6$ acyl groups, unsubstituted or oxo-substituted heterocyclic-CO- groups, adamantyl group, $C_1$-$C_6$ alkylsulfonyl groups and arylsulfonyl groups, provided that there are excluded the compounds in which $R^1$ represents an unsubstituted or $C_1$-$C_6$ alkyl-substituted phenyl group and $R^6$ represents a di-lower alkylamino group,

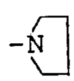 , 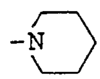 or 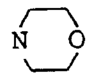 ;

all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo-[2,3-a] pyridyl, benzo[b]piperazinyl, chromenyl, isothiazolyl, isoxazolyl, oxadiazolyl, pyridazinyl, isoindolyl and isoquinolyl groups.

Incidentally, the cerebral function-improving agent mentioned herein refers to a cerebral function-improving agent having not only effects possessed by conventional cerebral function-improving agents, for example, sequelae of ischemic encephalopathy and cerebral apoplexy but also therapeutic or prophylactic effects for amnesia and dementias (e.g. cerebrovascular dementia, senile dementia and Alzheimer's dementia).

In the present specification, the following terms have the following definitions unless otherwise specified.

The term "halogen atom" means, for example, a fluorine atom, a chlorine atom, a bromine atom and an iodine atom; the term "$C_1$-$C_6$ alkyl group" means $C_{1-6}$ alkyl groups such as methyl, ethyl, n-propyl, isopropyl, n-butyl, isobutyl, tert-butyl, pentyl, hexyl and the like; the term "$C_1$-$C_6$ alkoxy group" means $C_{1-6}$ alkyl-O- groups; the term "$C_1$-$C_6$ alkylthio group" means $C_{1-6}$ alkyl-S-groups; the term "$C_2$-$C_6$ alkenyl group" means $C_{2-6}$alkenyl groups such as vinyl, propenyl, butenyl, pentenyl, hexenyl and the like; the term "$C_2$-$C_6$ alkenyloxy group" means $C_{2-6}$alkenyl-O- groups; the term "cycloalkyl group" means $C_{3-6}$cycloalkyl groups such as cyclopropyl, cyclobutyl, cyclopentyl, cyclohexyl and the like; the term "aryl group" means phenyl, naphthyl, indanyl and indenyl groups; the term "aryloxy group" means aryl-O- groups; the term "ar-$C_1$-$C_4$ alkyl group" means ar-$C_{1-4}$ alkyl groups such as benzyl, diphenylmethyl, trityl, phenethyl and the like; the term "ar-$C_1$-$C_4$ alkoxy group" means ar-$C_{1-4}$alkyl-O- groups; the term "ar-$C_1$-$C_4$ alkylthio group" means aryl-$C_{1-4}$alkyl-S- groups; the term "$C_1$-$C_4$ alkylenedioxy group" means $C_{1-4}$alkylenedioxy groups such as methylenedioxy, ethylenedioxy and the like; the term "$C_1$-$C_6$ acyl group" means $C_{1-6}$acyl groups such as formyl, acetyl, butyryl and the like; the term "aroyl group" means aryl-CO- groups; the term "$C_1$-$C_6$ alkylsulfonyl group" means $C_{1-6}$alkyl-$SO_2$- groups; the term "arylsulfonyl group" means aryl-$SO_2$- groups; ther term "ar-$C_1$-$C_6$ alkylsulfonyl group" means aryl-$C_{1-6}$alkyl-$SO_2$- groups; the term "$C_1$-$C_6$ alkylsulfonyloxy group" means $C_{1-6}$alkyl-$SO_2$-O- groups; the term "aryl-sulfonyloxy group" means aryl-$SO_2$-O- groups; the term "$C_1$-$C_6$ alkylsulfonylamino group" means $C_{1-6}$ alkyl-$SO_2$-NH-groups; the term "arylsulfonylamino group" means aryl-$SO_2$NH- groups; the term "di-$C_1$-$C_6$ alkylamino group" means di-$C_{1-6}$alkyl-NH- groups and the term "ammonio group" means tri-$C_1$-$C_6$ alkylammonio groups such as trimethylammonio, triethylammonio; the term "$C_1$-$C_6$ alkoxycarbonyl group" means $C_{1-6}$alkyl-O-CO- groups and the like.

The protective groups for hydroxyl group, carboxyl group and amino group include those conventional protective groups for hydroxyl group, carboxyl group and amino group which are described in Protective Groups in Organic Synthesis [Theodra W. Greene (1981), John Wiley & Sons, Inc.]. In particular, the protective group for hydroxyl group specifically includes, for example, lower alkyl, lower acyl, tetrahydropyranyl and ar-lower alkyl groups such as substituted or unsubstituted benzyl.

The salt of the 1,2-ethanediol derivative represented by the general formula [I] can be any pharmaceutically acceptable salt. It includes, for example, salts with mineral acids such as hydrochloric acid, hydrobromic acid, sulfuric acid, phosphoric acid and the like; salts with carboxylic acids such as formic acid, acetic acid, oxalic acid, fumaric acid, maleic acid, malic acid, tartaric acid, aspartic acid and the like; salts with sulfonic acids such as methanesulfonic acid, benzenesulfonic acid, p-toluenesulfonic acid, naphthalene-sulfonic acid and the like; and salts with alkali metals such as sodium, potassium and the like.

When the 1,2-ethanediol derivative of the general formula [I] or its salt has isomers (e.g. optical isomers, geometrical isomers, tautomers), all of these isomers are included in this invention. Also, the hydrate, solvate and all crystal forms of the compound of this invention are included in this invention.

Next, there is described a process for producing a 1,2-ethanediol derivative of the general formula [I] or a salt thereof.

The 1,2-ethanediol derivative of the general formula [I] or its salt can be produced by per se known processes or their appropriate combinations, for example, the following production processes.

Production Process 1

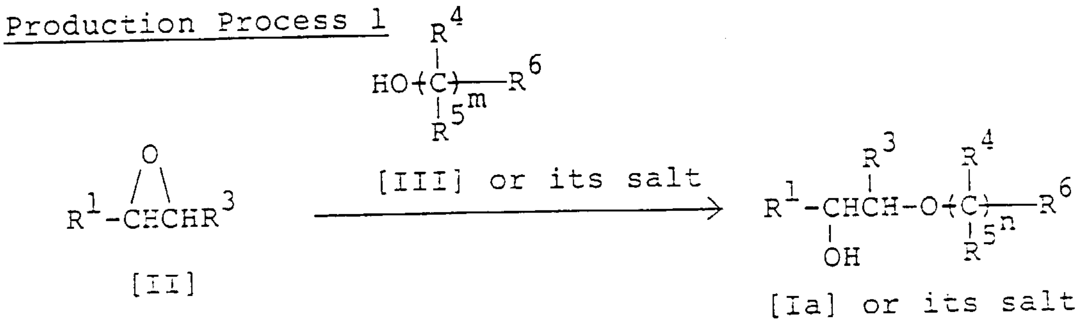


[II]

[Ia] or its salt

Production Process 2

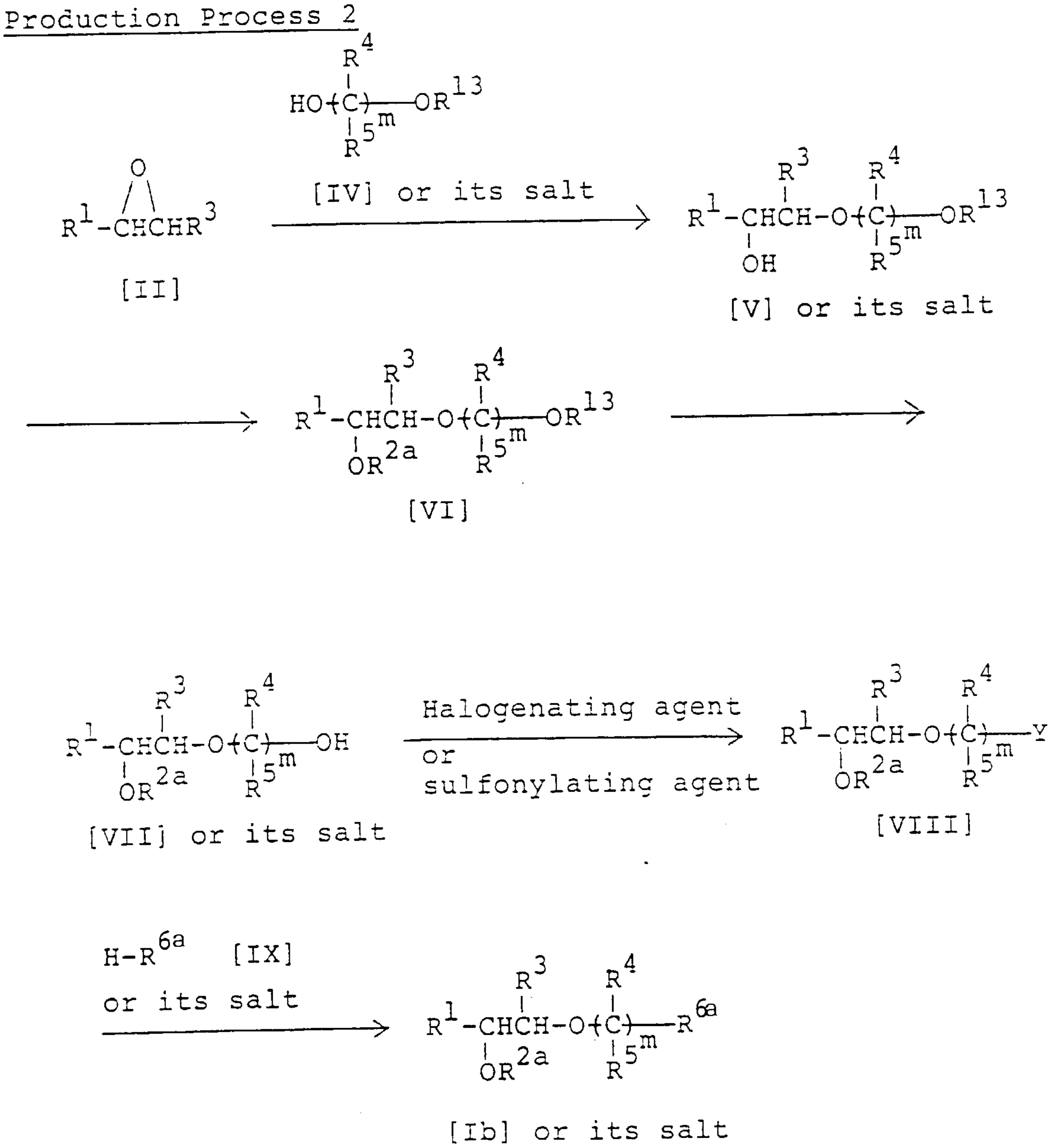


[II]

[V] or its salt

[VI]

[VII] or its salt

[VIII]

[Ib] or its salt

Production Process 3

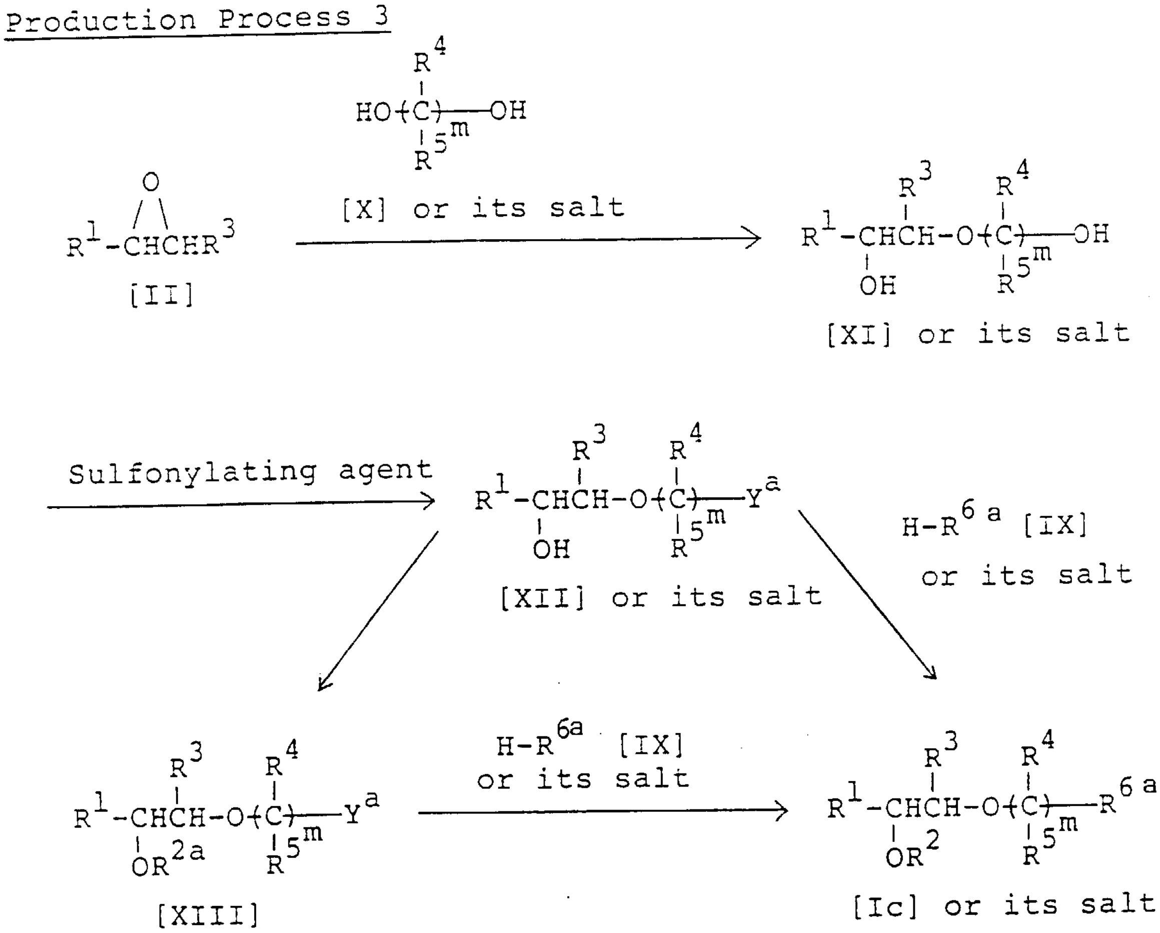


Production Process 4

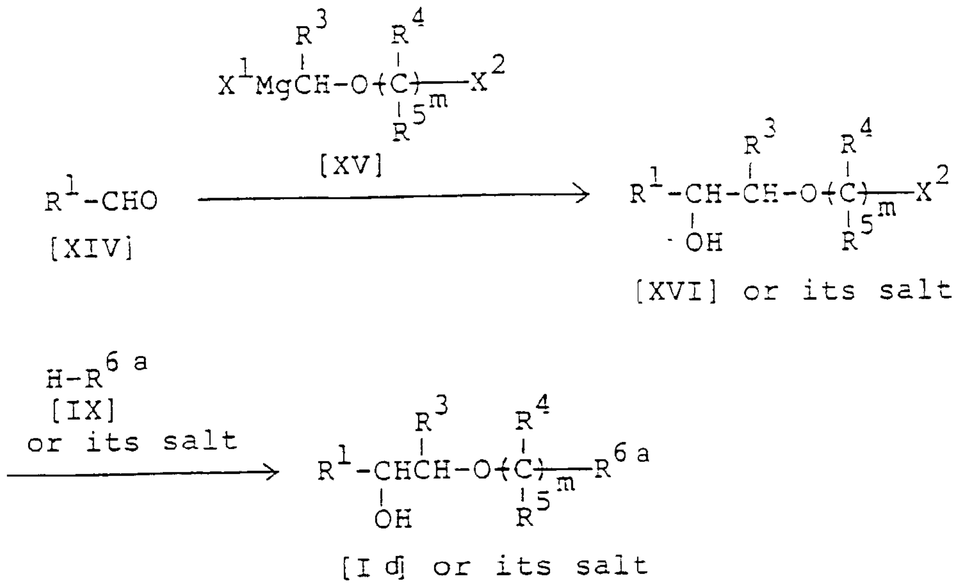

In the above reaction schemes, $R^1$, $R^2$, $R^3$, $R^4$, $R^5$, $R^6$ and n have the same meanings as defined above; $R^{2a}$ represents the same hydroxyl-protecting group as in the definition of $R^2$; $R^{6a}$ represents the same substituted or unsubstituted nitrogen-containing heterocyclic group as in the definition of $R^6$, provided that the nitrogen-containing heterocyclic group has a free valence on a nitrogen atom forming the heterocyclic ring, or a substituted or unsubstituted amino group; $R^{13}$ represents the same hydroxyl-protecting group as in the definition of $R^2$; $X^1$ and $X^2$, which may be the same or different, represent halogen atoms, Y represents a removable group such as a halogen atom, a $C_1$-$C_6$ alkyl-sulfonyloxy group, an arylsulfonyloxy group or the like; $Y^a$ represents an arylsulfonyloxy group; and m represents an integer of 1-6.

As the salts of the compounds of the general formulas [III], [IV], [V], [VII], [IX], [X], [XI], [XII], [XVI], [Ia], [Ib], [Ic] and [Id], there can be mentioned the same salts as the salts of the compound of the general formula [I].

Next, each of the above production processes is described.

Production Process 1

A compound of the general formula [II] is reacted with a compound of the general formula [III] or its salt in the presence or absence of a base to obtain a compound of the general formula [Ia] or its salt.

The solvent to be used in this reaction can be any solvent unless it adversely affects the reaction. There can be mentioned, for example, aromatic hydrocarbons such as benzene, toluene, xylene and the like; sulfoxides such as dimethylsulfoxide and the like; amides such as N,N-dimethylformamide and the like; and ethers such as tetrahydrofuran, dioxane and the like. These solvents can be used alone or in admixture of two or more. It is also possible to use the compound of the general formula [III as the solvent.

As the base to be used optionally, there can be mentioned, for example, sodium hydride, metallic sodium and potassium tert-butoxide.

In the reaction, the compound of the general formula [III] or its salt is used in an amount of 1-100 moles, preferably 1-10 moles, per mole of the compound of the general formula [II].

The base as an optional component is used in an amount of 0.01-1.2 moles per mole of the compound of the general formula [II].

This reaction can be effected usually at 20-150°C, preferably 70-90°C, for 1 minutes to 24 hours, preferably 5 minutes to 5 hours.

Production Process 2

(1) A compound of the general formula [II] is reacted with a compound of the general formula [IV] or its salt in the presence or absence of a base to obtain a compound of the general formula [V] or its salt.

This reaction can be effected in the same manner as described in Production Process 1.

The obtained compound of the general formula [V] or its salt may be used in the subsequent reaction without being isolated.

(2) The compound of the general formula [V] or its salt is subjected to conventional reaction for protection of hydroxyl group to obtain a compound of the general formula [VI].

The obtained compound of the general formula [VI] may be used in the subsequent reaction without being isolated.

Then, the compound of the general formula [VI] is subjected to reaction for selective removal of the hydroxyl-protecting group to obtain a compound of the general formula [VII] or its salt.

The obtained compound of the general formula [VII] or its salt may be used in the subsequent reaction without being isolated.

These reactions can be effected according to per se known methods, for example, the method described in Protective Groups in Organic Synthesis [Theodra W. Green (1981), John Wiley & Sons, Inc.] or a method similar thereto.

The combination of the hydroxyl-protecting groups ($R^{13}$ and $R^{2a}$) to be used in these reactions can be selected appropriately.

(3) The compound of the general formula [VII] or its salt is reacted with a halogenating agent or a sulfonylating agent in a solvent in the presence or absence of a base to obtain a compound of the general formula [VIII].

The solvent to be used in the reaction can be any solvent unless it adversely affects the reaction. There can be mentioned, for example, halogenated hydrocarbons such as methylene chloride, chloroform and the like; ethers such as tetrahydrofuran, dioxane and the like; nitriles such as acetonitrile and the like; and amides such as N,N-dimethylformamide and the like. These solvents can be used alone or in admixture of two or more.

As the base to be used optionally, there can be mentioned, for example, organic and.inorganic bases such as triethylamine, diisopropylethylamine, 1,8-diazabicyclo-[5.4.0]undec-7-ene (DBU) , pyridine, potassium tert-butoxide, sodium carbonate, potassium carbonate, sodium hydride and the like.

As the halogenating agent, there can be mentioned, for example, phosphorus oxychloride, phosphorus oxybromide, phosphorus trichloride, phosphorus pentachloride, thionyl chloride and the like.

As the sulfonylating agent, there can be mentioned, for example, methanesulfonyl chloride, p-toluenesulfonyl chloride and the like.

Each of the halogenating agent, the sulfonylating agent and the base as an optional component is used in an amount of at least 1 mole, preferably 1-2 moles, per mole of the compound of the general formula [VII] or its salt.

This reaction can be effected usually at -10° to 100°C, preferably 0° to 40°C, for 10 minutes to 30 hours.

The obtained compound of the general formula [VIII] may be used as it is, in the subsequent reaction without being isolated.

(4) The compound of the general formula [VIII] is reacted with a compound of the general formula [IX] or its salt in the presence or absence of a catalyst in the presence or absence of a base to obtain a compound of the general formula [Ib] or its salt.

The solvent to be used in this reaction can be any solvent unless it adversely affects the reaction. There can be mentioned, for example, the same solvents as mentioned in (3) of Production Process 2.

As the catalyst to be used optionally, there can be mentioned, for example, potassium iodide, sodium iodide and the like.

The catalyst is used in an amount of 0.1-1 mole per mole of the compound of the general formula [VIII].

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [VIII].

This reaction can be effected usually at 10-150°C, preferably 20-100°C for 10 minutes to 20 hours.

Production Process 3

(1) A compound of the general formula [II] is reacted with a compound of the general formula [X] or its salt in the presence or absence of a base to obtain a compound of the general formula [XI] or its salt.

This reaction can be effected in accordance with the same method as mentioned in Production Process 1.

(2) The compound of the general formula [XI] or its salt is reacted with a sulfonylating agent in a solvent in the presence or absence of a base to obtain a compound of the general formula [XII] or its salt.

The solvent to be used in this reaction can be any solvent unless it adversely affects the reaction. There can be mentioned, for example, the same solvents as mentioned in (3) of Production Process 2.

As the base to be used optionally, there can be mentioned, for example, the same bases as mentioned in (3) of Production Process 2.

As the sulfonylating agent, there can be mentioned, for example, p-toluenesulfonyl chloride and the like.

Each of the sulfonylating agent and the base as an optional component is used in an amount of at least 0.95 mole, preferably 1-2 moles, per mole of the compound of the general formula [XI] or its salt.

This reaction can be effected usually at -10° to 100°C, preferably 0° to 40°C, for 10 minutes to 30 hours.

The obtained compound of the general formula [XII] or its salt may be used in the subsequent reaction without being isolated.

(3) The compound of the general formula [XII] or its salt is subjected to conventional reaction for protection of hydroxyl group to obtain a compound of the general formula [XIII].

The reaction can be effected in accordance with per se known methods, for example, the method described in Protective Groups in Organic Synthesis [Theodra W. Green (1981), John Wiley & Sons, Inc.] or a method similar thereto.

The obtained compound of the general formula [XIII] may be used in the subsequent reaction without being isolated.

(4) The compound of the general formula [XII] or its salt or the compound of the general formula [XIII] is reacted with a compound of the general formula [IX] or its salt in the presence or absence of a base to obtain a compound of the general formula [Ic] or its salt.

This reaction can be effected in the same method as described in (4) of Production Process 2.

Production Process 4

(1) A compound of the general formula [XIV] is reacted with a compound of the general formula [XV] to obtain a compound of the general formula [XVI] or its salt.

The solvent to be used in this reaction can be any solvent unless it adversely affects the reaction. There can be mentioned, for example, ethers such as diethyl ether, tetrahydrofuran, dioxane and the like; and aromatic hydrocarbons such as benzene, toluene and the like. These solvents can be used alone or in admixture of two or more.

In this reaction, the compound of the general formula [XV] is used in an amount of 0.8-100 moles, preferably 0.8-10 moles, per mole of the compound of the general formula [XIV].

This reaction can be effected usually at -78° to 100°C, preferably -78° to 50°C for 5 minutes to 24 hours.

The obtained compound of the general formula [XVI] or its salt may be used in the subsequent reaction without being isolated.

Incidentally, the compound of the general formula [XV] to be used in this reaction can be produced according to a per se known method, for example, the method described in Bull. Soc. Chim. Fr., 1967 (5), pp. 1533-40.

(2) The compound of the general formula [XVI] or its salt is reacted with a compound of the general formula [IX] or its salt in the presence or absence of a base to obtain a compound of the general formula [Id] or its salt.

The solvent to be used in this reaction can be any solvent unless it adversely affects the reaction. There can be mentioned, for example, halogenated hydrocarbons such as methylene chloride, chloroform and the like; ethers such as tetrahydrofuran, dioxane and the like; alcohols such as ethanol, propanol, butanol and the like; nitriles such as acetonitrile and the like; and amides such as N,N-dimethylformamide and the like. These solvents can be used alone or in admixture of two or more.

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [XVI] or its salt.

This reaction can be effected usually at 10-150°C, preferably 20-100°C, for 10 minutes to 20 hours.

In the above production processes, the reactants or base can also be used as solvent depending on the nature of the reactants or base.

In the above production processes, when the compounds of the general formulas [II], [III], [IV], [V], [VI], [VII], [VIII], [IX], [X], [XI], [XII], [XIII], [XIV], [XV] and [XVI], have isomers (e.g. optical isomers, geometrical isomers, tautomers), the compounds can be used in any isomer form. Further, the compounds can be used in a hydrate form, a solvate form or any crystal form.

When the compounds of the general formulas [II], [III], [IV], [V], [VI], [VII], [VIII], [IX], [XII], [XIII], [XIV], [XV], [XVI], [I], [Ia], [Ib], [Ic] and [Id] have a hydroxyl group, an amino group or a carboxyl group, it is possible to previously protect these groups with a conventional protective group and to remove, after the reaction, the protective group, if necessary, according to a per se known method.

The compound thus obtained may be subjected to conventional isolation and purification procedures such as column chromatography, crystallization, distillation, extraction and the like.

The 1,2-ethanediol derivative of the general formula [I] or its salt can be converted into other 1,2-ethanediol derivative of the general formula [I] or its salt by subjecting the former compound to appropriate combination of per se known reactions such as oxidation reaction, reduction reaction, addition reaction, acylation reaction, alkylation reaction, sulfonylation reaction, deacylation reaction, substitution reaction, dehydration reaction, hydrolysis reaction and the like.

The compound of the general formula [II] which is the starting material for producing the compound of this invention can be produced by per se known processes, for example, the process described in JACS, vol. 87, p.1353 (1965) and the process described in Shin Jikken Kagaku Koza, vol. 14, p. 579 (1977), Maruzen.

The compound of this invention, when used as a drug, may be appropriately mixed with excipients such as filler, carrier, diluent and the like and can be formed into tablets, capsules, powders, granules, fine granules, pills, suspensions, emulsions, liquids, syrups, injections, etc. according to conventional methods. These drugs can be administered orally or parenterally. The dosage route, dose and number of administrations can be appropriately varied depending upon the age, weight and symptom of patient, but in the case of oral administration, generally 0.01-500 mg of the present compound can be administered daily to an adult patient in one to several portions.

Next, the pharmacological activities of representative compounds of this invention are described.

The numbers of the test compounds used in the following pharmacological tests refer to the numbers of the compounds shown in Production Examples appearing hereinafter.

1. Effect of test compound on hypoxia

A test compound dissolved in physiological saline (100 mg/kg) was orally administered to a ddY female mouse (5-6 weeks old, each group consisting of 10 mice). After 1 (or 30 minutes*) hour from the administration, each mouse was placed in a 300-ml glass chamber, and a gas mixture consisting of 4% of oxygen and 96% of nitrogen was passed through the chamber at a rate of 5 liters/min. A time from the start of gas passing to the death of each mouse was measured.

To a control mice group was orally administered only physiological saline.

The antihypoxic activity of the test compound was calculated from the following formula:

$$\frac{\text{Mean survival time of mice of administration group}}{\text{Mean survival time of mice of control group}} \times 100\ (\%)$$

The results are shown in Table 1.

Table 1

| Compound No. | Antihypoxic activity (%) | Compound No. | Antihypoxic activity (%) |
|---|---|---|---|
| 1 | 155 | 48 | 160 |
| 2 | 119 | 49 | 151 |
| 3 | 245* | 54 | 137 |
| 9 | 113 | 55 | 207 |
| 14 | 184* | 56 | 182 |
| 15 | 194* | 58 | 133 |
| 16 | 116 | 64 | 247* |
| 19 | 168* | 68 | 147 |
| 23 | 241* | 79 | 177* |
| 27 | 201* | 80 | 175* |
| 33 | 224* | 82 | 130* |
| 34 | 221 | 83 | 179 |
| 36 | 139 | 85 | 156* |
| 37 | 148 | 88 | 169 |
| 42 | 194 | 91 | 164 |
| 46 | 150* | | |
| 47 | 165* | | |

- To be cont'd -

Table 1 (Cont'd)

| Compound No. | Antihypoxic activity (%) |
|---|---|
| 92 | 176* |
| 93 | 138* |
| 94 | 164* |
| 96 | 171* |
| 98 | 140* |
| 100 | 160* |
| 118 | 165 |
| 130 | 176* |
| 131 | 132 |
| 137 | 153* |
| 144 | 176* |
| 146 | 164* |
| 152 | 177* |
| 153 | 176* |
| 156 | 136* |
| 157 | 242* |
| 158 | 148* |
| 160 | 177* |
| 162 | 149 |
| 164 | 152* |
| 165 | 149* |
| 166 | 155 |
| 167 | 157* |

Note: * Mice were placed in the chamber after 30 minutes from the administration instead of after 1 hr from the administration.

2. Effect of test compound on amnesia

(1) Electroconvulsive shock (ECS)-induced amnesia

A test compound dissolved in physiological saline was intraperitoneally administered to a ddY male mouse (5-6 weeks old, each group consisting of 10 mice). The acquisition trial in passive avoidance task was carried out after 1 hour from the administration. Each mouse was placed in the bright compartment of a two-compartment step-through type passive avoidance apparatus consisting of a bright compartment and a dark compartment (MPA-100M manufactured by Muromachi Kikai). When the mouse entered the dark compartment, the guilotine door of the dark compartment was closed; after 0.5 second, an inescapable footshock (1.6 mA, 3 seconds) was delivered. Immediately thereafter, ECS (25 mA, 0.5 second) was applied through the both eyes of the mouse. After 24 hours, in the retention trial, the mouse was again placed in the bright compartment and the response latency for mouse to enter the dark compartment was measured. If the mouse avoided longer than 300 seconds, a ceiling score of 300 seconds was assigned.

A control mice group to which only physiological saline had been administered intraperitoneally, and response latency was also measured in the same manner.

Antiamnesic activity was taken as a median of the response latencies of the 10 mice and expressed by the following symbols:

- : 0-60 seconds
+ : 61-100 seconds
++ : 101-150 seconds
+++ : 151-300 seconds

The results are shown in Table 2.

Table 2

| Compound No. | Dosage (mg/Kg) | Antiamnesic activity |
|---|---|---|
| 2 | 3 | + |
| 3 | 3 | +++ |
| 8 | 3 | + |
| 15 | 3 | +++ |
| 19 | 3 | + |
| 23 | 10 | + |
| 29 | 3 | + |
| 44 | 10 | ++ |
| 48 | 3 | ++ |
| 52 | 3 | + |
| 53 | 3 | +++ |
| 58 | 10 | ++ |
| 64 | 3 | ++ |
| 67 | 3 | ++ |
| 73 | 3 | ++ |
| 110 | 3 | + |
| 111 | 3 | ++ |
| 121 | 3 | ++ |
| 138 | 3 | +++ |

(2) Effect of test compound on cycloheximide-induced amnesia

It was reported by Yamazaki et al. [Drugs, Mind and Action, vol. 3, pp. 127-136 (1983)] that cycloheximide interfers with the retrieval process of memory of mouse. Hence, the following test was carried out.

A test was carried out in accordance with the method described in Drugs, Mind and Action, vol. 3, pp. 127-136 (1983) and Folia Pharmacologica Japonica, vol. 89, pp. 243-252 (1987).

As the test apparatus, there was used a step-down type passive avoidance training box. It was a black acrylic resin box of 22 cm x 22 cm x 21 cm (height) whose floor portion consisted of a stainless steel grid and which had, at

one corner of the floor grid, a platform of 7 cm x 7 cm x 2 cm (height).

Cycloheximide was dissolved in physiological saline, and injected subcutaneously at 120 mg/kg to a ddY male mouse (5-6 weeks old, each group consisting of 10 mice). In an acquisition trial, after 15 minutes from the administration, each mouse was placed on the platform in the above test apparatus. As soon as the mouse stepped down the platform, a 2 mA current was delivered for 2 seconds, immediately after which the mouse was returned to its home cage. A retention test was performed 24 hours after the acquisition. To each mouse which had been treated with cycloheximide was orally administered a test compound dissolved in physiological saline; after 30 minutes from the administration, the mouse was again placed on the platform and the response latency for mouse to step down was measured. If the mouse avoided longer than 300 seconds, a ceiling score of 300 seconds was assigned.

A control mice group to which only physiological saline had been administered orally, and response latency was also measured in the same manner.

Antiamnesic activity was taken as a median of the response latencies of the 10 mice and expressed by the following symbols:

- : 0-60 seconds<br>
+ : 61-100 seconds<br>
++ : 101-150 seconds<br>
+++ : 151-300 seconds

The results are shown in Table 3.

Table 3

| Compound No. | Dosage (mg/Kg) | Antiamnesic activity |
|---|---|---|
| 1 | 3 | ++ |
| 7 | 10 | + |
| 9 | 10 | + |
| 14 | 3 | +++ |
| 15 | 3 | +++ |
| 16 | 10 | + |
| 25 | 3 | + |
| 28 | 10 | + |
| 42 | 3 | + |
| 48 | 3 | ++ |
| 49 | 3 | ++ |
| 54 | 3 | ++ |
| 56 | 3 | + |
| 62 | 3 | ++ |
| 78 | 3 | ++ |
| 79 | 3 | + |
| 80 | 10 | ++ |
| 96 | 3 | ++ |
| 106 | 10 | ++ |
| 108 | 3 | +++ |
| 116 | 10 | + |
| 128 | 3 | +++ |
| 131 | 10 | ++ |
| 143 | 3 | + |
| 145 | 3 | +++ |
| Conrol | - | - |

3. Inhibitory activity for acetylcholinesterase

A test was conducted in accordance with the method of Ellman et al. [Biochem. Pharmacol., vol. 7, pp. 88-95, 1961]. That is, acetylthiocholine (as a substrate) was added to a phosphate buffer solution containing 5,5'-dithiobis-(2-nitrobenzoic acid) (DTNB), a test compound and a mouse cerebral homogenate (as an acetylcholinesterase source). The resulting mixture was incubated, and the amount of the resulting 5-thio-2-nitrobenzoic acid was photometrically measured at 412 nm.

The inhibitory activity for acetylcholinesterase of the test compound was expressed by the inhibition (%) when the final concentration of the test compound was 10 µg/ml.

The results are shown in Table 4.

Table 4

| Compound No. | Inhibition (%) | Compound No. | Inhibition (%) |
|---|---|---|---|
| 6 | 13 | 86 | 52 |
| 30 | 22 | 93 | 38 |
| 34 | 26 | 99 | 36 |
| 37 | 45 | 116 | 24 |
| 38 | 36 | 132 | 38 |
| 40 | 26 | 139 | 76 |
| 47 | 38 | 164 | 83 |
| 48 | 51 | 165 | 74 |
| 54 | 83 | 166 | 36 |
| 55 | 22 | 167 | 43 |
| 58 | 45 | | |
| 62 | 62 | | |
| 64 | 90 | | |
| 67 | 91 | | |
| 68 | 87 | | |
| 69 | 92 | | |
| 70 | 89 | | |
| 79 | 93 | | |
| 82 | 50 | | |
| 85 | 44 | | |

4. Acute toxicity

A test compound dissolved in physiological saline was intravenously administered to a group of 3 ddY male mice (5-6 weeks old) to examine the acute toxicity of the test compound.

As a result, test compound Nos. 1, 2, 3, 6, 8, 9, 15, 16, 25, 30, 33, 34, 38, 40, 42, 44, 46, 52, 53, 54, 62, 64, 67, 68, 108, 110, 111, 121, 128, 132, 139, 143 and 158 gave no death case at 50mg/kg.

It is easily appreciated from the above test results that the compound of this invention is excellent in antihypoxic activity, antiamnesic activity and inhibitory activity for acetylcholinesterase and has low toxicity.

From the above result, it is also easily appreciated that the cerebral function-improving agent of this invention is useful for treating cerebrovascular dementia, senile dementia, Alzheimer's dementia, sequelae of ischemic encephalopathy and cerebral apoplexy.

Next, the process for producing the compound of this invention is specifically described by way of Production Examples.

In the Production Examples, the mixing ratio of eluant is by volume in all cases and, as the carrier in column chromatography, there was used a silica gel (Kieselgel 60, Art. 7734) manufactured by Merck Co.

The abbreviations used in the Production Examples have the following meanings.

Me: methyl, Et: ethyl, i-Pr: isopropyl, t-Bu: tert-butyl, Ac: acetyl, Ph: phenyl, DPM: diphenylmethyl, Bz: benzyl, Tr: trityl, IPA: isopropyl alcohol, IPE: diisopropyl ether, PTS: p-toluenesulfonic acid.

In the following sentences and tables, the substances in [ ] refer to solvents used in recrystallization.

Compounds No. 1-8, 10, 12-24, 26-30, 86, 98, 100, 102, 104, 107, 130, 161 and 168 are outside the scope of compound claim 1 but within the scope of the invention defined in claims 5 to 11.

Production example 1

(1) A mixture of 10.8 g of (L)-dibenzoylcystine, 1.6 g of lithium borohydride, 2.4 g of tert-butanol and 180 ml of

tetrahydrofuran was refluxed for 1 hour and then cooled to -60°C. Thereto was added 6.1 g of 4-benzyloxyphenacyl bromide. The mixture was stirred for 30 minutes at the same temperature and further for 3 hours at -40° to -30°C. The reaction mixture was added to a mixture of 100 ml of water and 200 ml of diethyl ether. The organic layer was separated, washed with water, a saturated aqueous sodium hydrogencarbonate solution and a saturated aqueous sodium chloride solution in this order, and dried over anhydrous magnesium sulfate. The solvent was removed by distillation under reduced pressure. The residue thus obtained was purified by column chromatography (eluant: toluene) to obtain 2.8 g of (S)-1-(4-benzyloxyphenyl)-2-bromoethanol.

(2) 2.8 g of (S)-1-(4-benzyloxyphenyl)-2-bromoethanol was dissolved in a mixed solvent of 20 ml of methanol and 10 ml of tetrahydrofuran. To the solution was added a solution of 0.8 g of potassium hydroxide dissolved in 4 ml of water, with ice cooling. The mixture was stirred for 5 minutes at the same temperature and further for 10 minutes at room temperature. The reaction mixture was added to a mixture of 50 ml of diethyl ether and 50 ml of ice water. The organic layer was separated. The aqueous layer was extracted with 25 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure to obtain 1.4 g of (S)-2-(4-benzyloxyphenyl)oxirane.

Melting point: 56-61°C
Optical rotation: $[\alpha]_D^{25}$ = +5.2° (C=2, $CHCl_3$)

The following compounds were obtained in the same manner.

o(S)-2-(3-Methylphenyl)oxirane
$[\alpha]_D^{25}$ = +15.7° (C=2, $CHCl_3$)
o(S)-2-(4-Phenoxyphenyl)oxirane
$[\alpha]_D^{25}$= +12.5° (C=4, $CHCl_3$)

Production Example 2

(1) A solution of 23 g of (+)-diisopinocamphenylchloroborane dissolved in 30 ml of tetrahydrofuran was cooled to -25°C. Thereto was added 12 g of 4-benzyloxyphenacyl bromide. The resulting mixture was stirred for 4 hours at -20° to -15°C. The reaction mixture was added to a mixture of 150 ml of diethyl ether and 100 ml of ice water. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: hexane:toluene = 1:2) to obtain 6.8 g of (R)-1-(4-benzyloxyphenyl)-2-bromoethanol.

(2) 6.0 of (R)-1-(4-benzyloxyphenyl)-2-bromoethanol was dissolved in a mixed solvent of 50 ml of methanol and 25 ml of tetrahydrofuran. Thereto was added a solution of 1.5 g of potassium hydroxide dissolved in 5 ml of water, with ice cooling. The resulting mixture was stirred for 5 minutes at the same temperature and further for 10 minutes at room temperature. The reaction mixture was added to a mixture of 100 ml of diethyl ether and 100 ml of ice water. The organic layer was separated. The aqueous layer was extracted with 50 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure to obtain 3.7 g of (R)-2-(4-benzyloxyphenyl)oxirane.

Melting point: 58-67°C
Optical rotation: $[\alpha]_D^{26}$ = -14.4° (C=2, $CHCl_3$)

The following compounds were obtained in the same manner.

o(R)-2-(3-Methylphenyl)oxirane
$[\alpha]_D^{27}$ = -18.6° (C=2, $CHCl_3$)
o(R)-2-(4-Phenoxyphenyl)oxirane
$[\alpha]_D^{25}$ = -11.3° (C=2, $CHCl_3$)

Production Example 3

3.4 of potassium tert-butoxide was added to 31 ml of 2-(N,N-dimethylamino)ethanol. The resulting mixture was heated to 80°C. Thereto was dropwise added 7.7 g of 2-(3-fluorophenyl)oxirane over 40 minutes. The mixture was stirred for 3 hours at the same temperature. The reaction mixture was added to a mixture of 200 ml of ice water and 200 ml of ethyl acetate. The organic layer was separated. To the organic layer was added 50 ml of water. The mixture was adjusted to pH 1 with 6 N hydrochloric acid. The aqueous layer was separated and mixed with 100 ml of chloroform. The resulting mixture was adjusted to pH 11 with a 2 N aqueous sodium hydroxide solution. The organic layer was

separated, washed with water, and dried over anhydrous magnesium sulfate. The solvent was removed by distillation under reduced pressure. The residue thus obtained was purified by column chromatography (eluant: chloroform/ethanol = 5/1). The resulting oily product was dissolved in 25 ml of acetone. Hydrogen chloride gas was blown into the solution. The resulting crystals were collected by filtration, washed with acetone and dried to obtain 3.1 g of 2-[2-(N,N-dimethylamino)ethoxy]-1-(3-fluorophenyl)ethanol hydrochloride (compound No. 1).

Melting point: 164-165°C [EtOH]

The compounds shown in Table 5 were obtained in the same manner.

In Table 5, $R^1$, $R^2$, $R^3$, $R^{4a}$, $R^{4b}$, $R^6$, na and nb each show a substituent or integer used in the following formula.

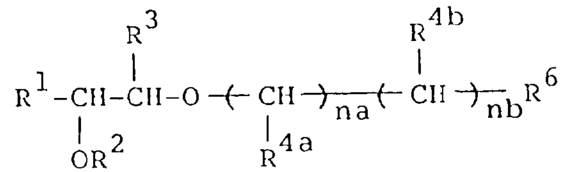

Table 5

| Compound No. | $R^1$ | $R^2$ | $R^3$ | $R^{4a}$ | $R^{4b}$ | $R^6$ | na | nb | Addition salt | Melting point (°C) |
|---|---|---|---|---|---|---|---|---|---|---|
| 2 | 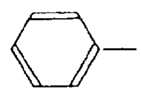 | H | H | H | H | 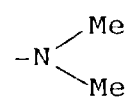 | 1 | 1 | HCl | 184-185 [EtOH] |
| $3^{*a}$ | " (R-form) | " | " | " | " | " | " | " | " | 174.5-175 [$Me_2CO$-EtOH] |

Table 5 (cont'd)

| | | | | | | | | | | |
|---|---|---|---|---|---|---|---|---|---|---|
| 4*b | 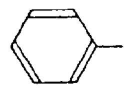 (S-form) | H | H | H | H | 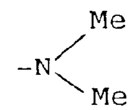 | 1 | 1 | HCl | 174.5-175 [$Me_2CO$-EtOH] |
| 5 | 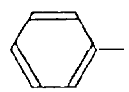 | " | " | " | " | 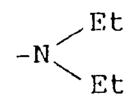 | " | " | - | Oily |
| 6 | " | " | " | " | " | 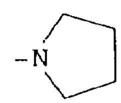 | " | " | HCl | 173-174 [EtOH-$Et_2O$] |
| 7 | " | " | " | " | " | 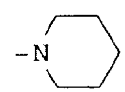 | " | " | " | 163.5-164.5 [EtOH-$Et_2O$] |

Table 5 (cont'd)

| | | | | | | | | | | |
|---|---|---|---|---|---|---|---|---|---|---|
| 8 | 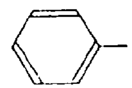 | H | H | H | H | 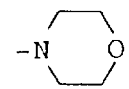 | 1 | 1 | HCl | 153-154.5 [EtOH-$Et_2O$] |
| 9 | " | " | " | " | " | 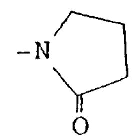 | " | " | - | Oily |
| 10 | " | " | " | " | " | 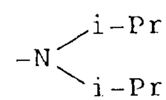 | " | " | " | " |
| 11 | " | " | " | " | " | 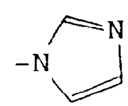 | " | " | " | " |

Table 5 (cont'd)

| | | | | | | | | | | |
|---|---|---|---|---|---|---|---|---|---|---|
| 12 | 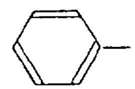 | H | H | H | H | 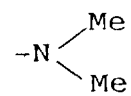 | 2 | 1 | - | Oily |
| 13 | " | " | " | " | " | " | 3 | 3 | HCl | Amorphous |
| 14 | 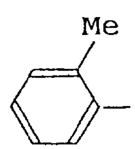 | " | " | " | " | " | 1 | 1 | " | 198-199 [EtOH] |
| 15 | 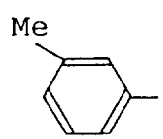 | " | " | " | " | " | " | " | " | 165-166 [IPA] |

Table 5 (cont'd)

| | | | | | | | | | | |
|---|---|---|---|---|---|---|---|---|---|---|
| 16 | 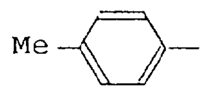 | H | H | H | H | 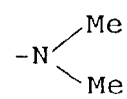 | 1 | 1 | HCl | 181.5-183 [EtOH] |
| 17 | 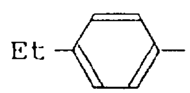 | " | " | " | " | " | " | " | " | 201.5-203 [IPA] |
| 18 | 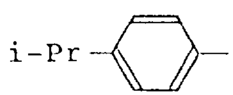 | " | " | " | " | " | " | " | " | 194.5-196.5 [EtOH] |
| 19 | 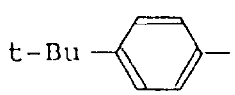 | " | " | " | " | " | " | " | " | 251-252 [EtOH] |

Table 5 (cont'd)

| | | | | | | | | | | |
|---|---|---|---|---|---|---|---|---|---|---|
| 20 | 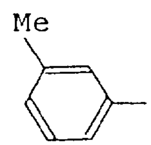 | H | H | H | H | 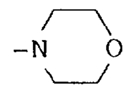 | 1 | 1 | HCl | 141-143 [EtOH-$Et_2O$] |
| 21 | " | " | " | " | " | 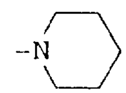 | " | " | " | 136.5-137.5 [EtOH-$Et_2O$] |
| 22 | " | " | " | " | " | 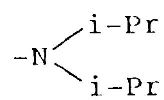 | " | " | - | Oily |
| 23 | " | " | " | " | " | 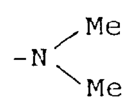 | 2 | 2 | " | " |

Table 5 (cont'd)

| | | | | | | | | | | |
|---|---|---|---|---|---|---|---|---|---|---|
| 24 | 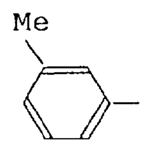 | H | H | H | H | 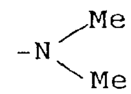 | 1 | 2 | - | Oily |
| 25 | " | " | " | " | " | 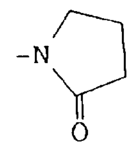 | " | 1 | " | ". |
| 26 | 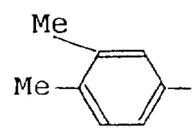 | " | " | " | " | 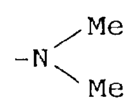 | " | " | HCl | 184-185 [EtOH] |
| 27 | 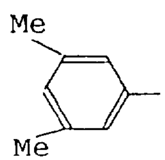 | " | " | " | " | " | " | " | " | 158-159 [IPA] |

Table 5 (cont'd)

| | | | | | | | | | | |
|---|---|---|---|---|---|---|---|---|---|---|
| 28 | 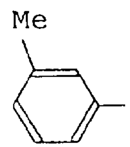 | H | H | Me | H | 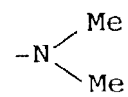 | 1 | 1 | - | Oily |
| 29 | " | " | " | H | Me | " | " | " | " | " |
| 30 | 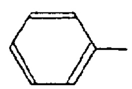 | " | Me | " | H | " | " | " | HCl | 190-191 [MeOH-$Et_2O$] |
| 31 | 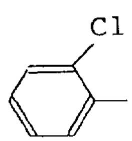 | " | H | " | " | " | " | " | " | 203-204 [EtOH] |

Table 5 (cont'd)

| | | | | | | | | | | |
|---|---|---|---|---|---|---|---|---|---|---|
| 32 | 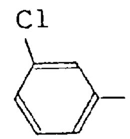 | H | H | H | H | 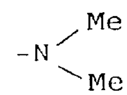 | 1 | 1 | HCl | 170-171 [$Me_2CO$] |
| 33 | 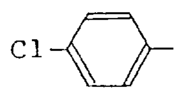 | " | " | " | " | " | " | " | " | 188.5-190 [MeOH-$Et_2O$] |
| 34 | 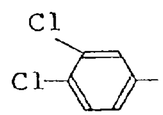 | " | " | " | " | " | " | " | " | 156.5-157.5 [EtOH-$Et_2O$] |
| 35 | 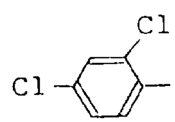 | " | " | " | " | " | " | " | " | 184-184.5 [EtOH] |

Table 5 (cont'd)

| | | | | | | | | | | |
|---|---|---|---|---|---|---|---|---|---|---|
| 36 | 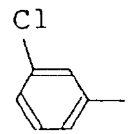 | H | H | H | H | 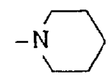 | 1 | 1 | HCl | 131-132 [EtOH-$Et_2O$] |
| 37 | 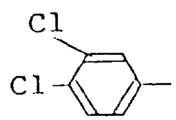 | " | " | " | " | " | " | " | " | 178.5-179.5 [EtOH-$Et_2O$] |
| 38 | 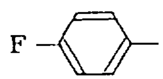 | " | " | " | " | 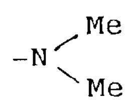 | " | " | " | 171-172 [EtOH] |
| 39 | 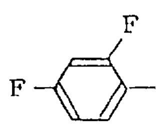 | " | " | " | " | " | " | " | " | 182-182.5 [EtOH] |

Table 5 (cont'd)

| | | | | | | | | | | |
|---|---|---|---|---|---|---|---|---|---|---|
| 40 | 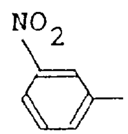 | H | H | H | H | 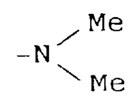 | 1 | 1 | HCl | 228-229.5 [EtOH-$Et_2O$] |
| 41 | 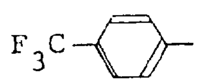 | " | " | " | " | " | " | " | " | 193.5-194.5 [EtOH] |
| 42 | 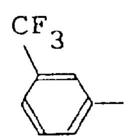 | " | " | " | " | " | " | " | " | 168.5-169.5 [EtOH-$Et_2O$] |
| 43 | 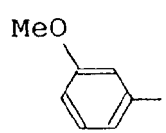 | " | " | " | " | " | " | " | " | 146-147 [EtOH] |

Table 5 (cont'd)

| | | | | | | | | | | |
|---|---|---|---|---|---|---|---|---|---|---|
| 44 | 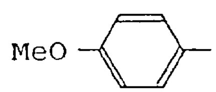 | H | H | H | H | 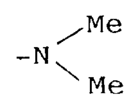 | 1 | 1 | HCl | 171-173 [EtOH] |
| 45 | 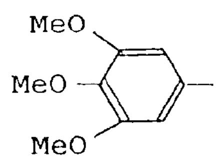 | " | " | " | " | " | " | " | PTS | 140-141 [EtOH] |
| 46 | 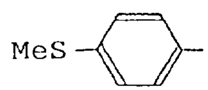 | " | " | " | " | " | " | " | HCl | 179.5-181.5 [EtOH-$Et_2O$] |
| 47 | 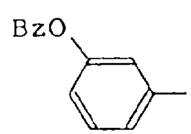 | " | " | " | " | " | " | " | " | 169-171 [EtOH] |

Table 5 (cont'd)

| | | | | | | | | | | |
|---|---|---|---|---|---|---|---|---|---|---|
| 48 | 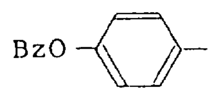 | H | H | H | H | 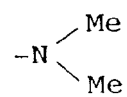 | 1 | 1 | HCl | 186-186.5 [EtOH] |
| 49 | 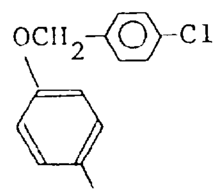 | " | " | " | " | " | " | " | " | 188-188.5 [EtOH] |
| 50 | 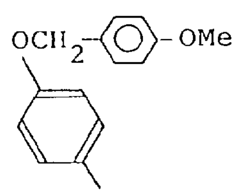 | " | " | " | " | " | " | " | " | 199-199.5 [MeOH-EtOH] |
| 51 | 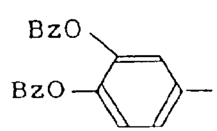 | " | " | " | " | " | " | " | - | Oily |

Table 5 (cont'd)

| | | | | | | | | | | |
|---|---|---|---|---|---|---|---|---|---|---|
| 52 | 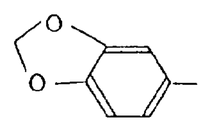 | H | H | H | H | 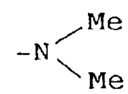 | 1 | 1 | HCl | 149.5-151 [$Me_2CO$] |
| 53 | 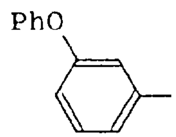 | " | " | " | " | " | " | " | " | 140-141 [EtOH-$Et_2O$] |
| 54 | 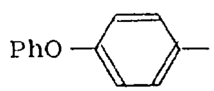 | " | " | " | " | " | " | " | " | 174.5-176.5 [MeCN] |
| 55 | 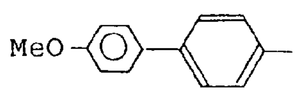 | " | " | " | " | " | " | " | " | 229-231 [MeCN] |

Table 5 (cont'd)

| | | | | | | | | | | |
|---|---|---|---|---|---|---|---|---|---|---|
| 56 | 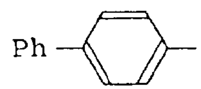 | H | H | H | H | 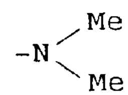 | 1 | 1 | HCl | 217.5-218.5 [EtOH] |
| 57 | 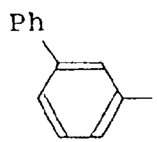 | " | " | " | " | " | " | " | " | 138-140 [EtOH-$Et_2O$] |
| 58 | 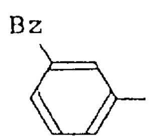 | " | " | " | " | " | " | " | " | 153-154 [EtOH] |
| 59 | 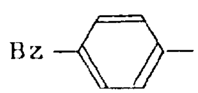 | " | " | " | " | " | " | " | " | 179-181 [EtOH] |

Table 5 (cont'd)

| | | | | | | | | | | |
|---|---|---|---|---|---|---|---|---|---|---|
| 60 | 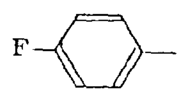 | " | " | " | " | 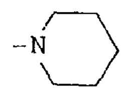 | " | " | HCl | 160-161 [EtOH-$Et_2O$] |

Table 5 (cont'd)

| | | | | | | | | | | |
|---|---|---|---|---|---|---|---|---|---|---|
| 61 | 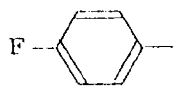 | H | H | H | H | 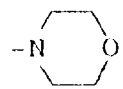 | 1 | 1 | HCl | 151-152.5 [EtOH-$Et_2O$] |
| 62 | 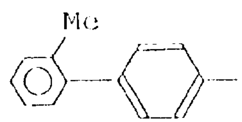 | " | " | " | " | 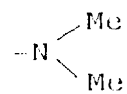 | " | " | " | 223-225 [EtOH-IPA] |
| 63 | 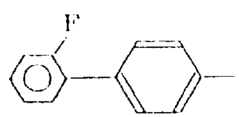 | " | " | " | " | " | " | " | " | 215-216 [MeOH-EtOH] |
| 64 | 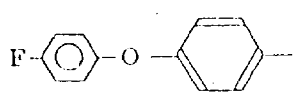 | " | " | " | " | " | " | " | " | 180.5-181.5 [MeCN] |

Table 5 (cont'd)

| | | | | | | | | | | |
|---|---|---|---|---|---|---|---|---|---|---|
| 65 | 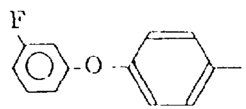 | H | H | H | H | 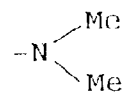 | 1 | 1 | HCl | 168.5-169.5 [IPA] |
| 66 | 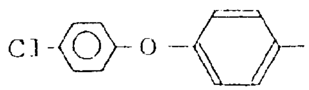 | " | " | " | " | " | " | " | " | 177.5-178.5 [MeCN] |
| 67 | 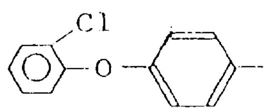 | " | " | " | " | " | " | " | " | 194.5-195 [MeCN] |
| 68 | 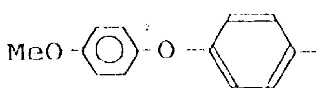 | " | " | " | " | " | " | " | " | 162-162.5 [IPA] |

Table 5 (cont'd)

| | | | | | | | | | | |
|---|---|---|---|---|---|---|---|---|---|---|
| 69 | 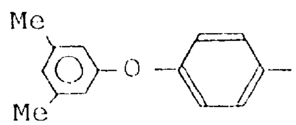 | H | H | H | H | 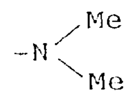 | 1 | 1 | HCl | 171-172 [IPA] |
| 70 | 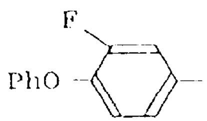 | " | " | " | " | " | " | " | " | 162-162.5 [IPA] |
| 71 | 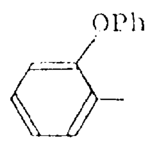 | " | " | " | " | " | " | " | " | 147.5-148.5 [$Me_2CO$-$Et_2O$] |
| 72 | 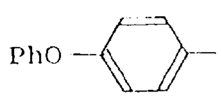 | " | " | " | " | " | 2 | " | - | Oily |

Table 5 (cont'd)

| | | | | | | | | | | |
|---|---|---|---|---|---|---|---|---|---|---|
| 73 | 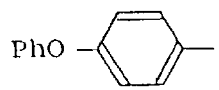 | H | H | H | H | 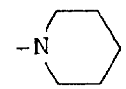 | 1 | 1 | HCl | 160-160.5 [IPA] |
| 74 | " | " | " | " | " | 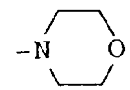 | " | " | " | 192.5-193 [MeCN] |
| 75 | 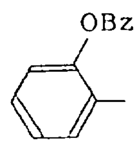 | " | " | " | " | 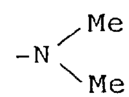 | " | " | HCl | 124-125 [MeCN] |

Table 5 (cont'd)

| | | | | | | | | | | |
|---|---|---|---|---|---|---|---|---|---|---|
| 76 | 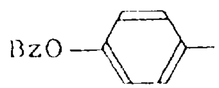 | H | H | H | H | 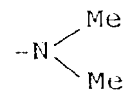 | 2 | 1 | - | 121.5-123 [AcOEt-$Et_2O$] |
| 77 | 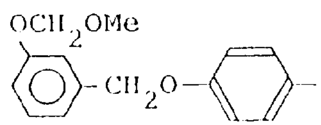 | " | " | " | " | " | 1 | " | " | Oily |
| 78*c | 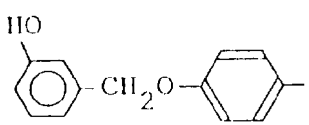 | " | " | " | " | " | " | " | HCl | 179-181 [EtOH] |
| 79 | 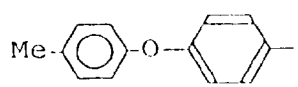 | " | " | " | " | " | " | " | " | 189.5-190 [IPA] |

Table 5 (cont'd)

| | | | | | | | | | | |
|---|---|---|---|---|---|---|---|---|---|---|
| 80 | 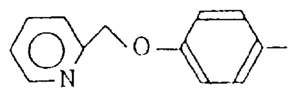 | H | H | H | H | 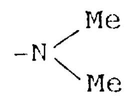 | 1 | 1 | 2HCl | 157-158 [EtOH] |
| 81 | 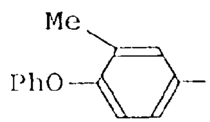 | " | " | " | " | " | " | " | HCl | 161-162.5 [IPA] |
| 82 | 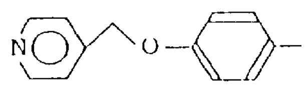 | " | " | " | " | " | " | " | 2HCl | 157.5-159.5 ·[EtOH] |
| 83 | 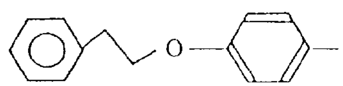 | " | " | " | " | " | " | " | HCl | 172-173 [IPA] |

Table 5 (cont'd)

| | | | | | | | | | | |
|---|---|---|---|---|---|---|---|---|---|---|
| 84 | 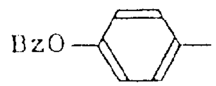 | H | H | H | H | 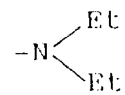 | 1 | 1 | - | Oily |
| 85 | 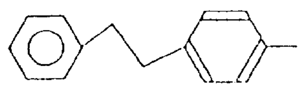 | " | " | " | " | 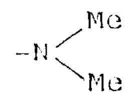 | " | " | HCl | 222.5-223.5 [EtOH] |
| 86 | 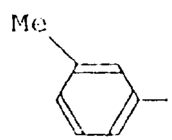 | " | " | " | " | 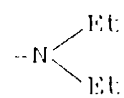 | " | " | - | Oily |
| 87 | 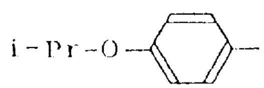 | " | " | " | " | 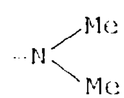 | " | " | HCl | 204.5-205 [EtOH-$Me_2CO$] |

Table 5 (cont'd)

| | | | | | | | | | | |
|---|---|---|---|---|---|---|---|---|---|---|
| 88 | 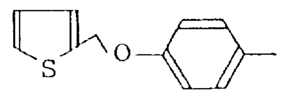 | H | H | H | H | 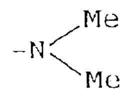 | 1 | 1 | HCl | 168.5-169 [EtOH-AcOEt] |
| 89 | 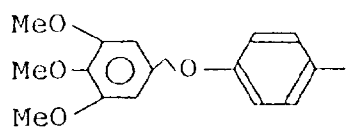 | " | " | " | " | " | " | " | " | 193-194 [EtOH-AcOEt] |
| 90 | 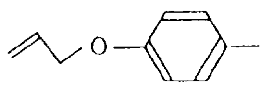 | " | " | " | " | " | " | " | " | 154.5-156 [MeCN] |
| 91 | 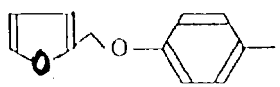 | " | " | " | " | " | " | " | " | 168.5-169.5 [EtOH-AcOEt] |

Table 5 (cont'd)

| | | | | | | | | | | |
|---|---|---|---|---|---|---|---|---|---|---|
| 92 | 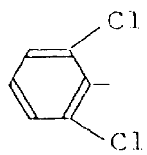 | H | H | H | H | 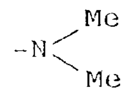 | 1 | 1 | HCl | 197-198 [IPA-AcOEt] |
| 93 | 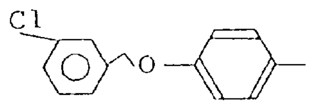 | " | " | " | " | " | " | " | " | 176-178 [EtOH] |
| 94 | 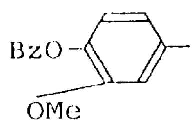 | " | " | " | " | " | " | " | " | 140.5-142 [IPA] |
| 95 | 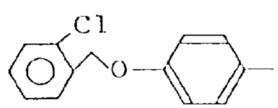 | " | " | " | " | " | " | " | " | 181.5-182.5 [EtOH] |

Table 5 (cont'd)

| | | | | | | | | | | |
|---|---|---|---|---|---|---|---|---|---|---|
| 96 | 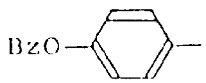 | H | H | H | H | 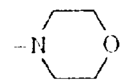 | 1 | 1 | HCl | 170-170.5 [EtOH-$Me_2CO$] |
| 97 | 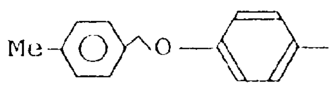 | " | " | " | " | 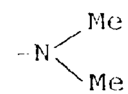 | " | " | " | 199-199.5 [EtOH] |
| 98 *d | 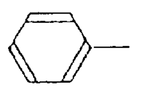 | " | " | " | " | $-NH_2$ | " | " | 1/2 Fumaric acid | 181-182.5 |
| 99 *e | 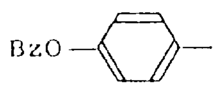 (R-form) | " | " | " | " | 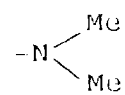 | " | " | HCl | 194-194.5 [EtOH-$Me_2CO$] |

Table 5 (cont'd)

| | | | | | | | | | | |
|---|---|---|---|---|---|---|---|---|---|---|
| 100 *f | 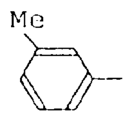 (R-form) | H | H | H | H | 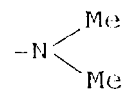 | 1 | 1 | HCl | 164-165 [EtOH-$Me_2CO$] |
| 101 *g | 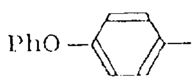 (R-form) | " | " | " | " | " | " | " | " | 193-194 [MeCN] |
| 102 *h | 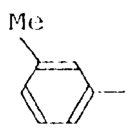 | " | " | " | " | 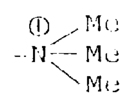 | " | " | 1/2 1,5-Naphthalenedisulfonic acid | Amorphous |
| 103 *i | 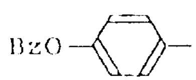 (S-form) | " | " | " | " | 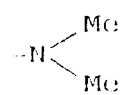 | " | " | HCl | 195-196 [EtOH-$Me_2CO$] |

Table 5 (cont'd)

| | | | | | | | | | | |
|---|---|---|---|---|---|---|---|---|---|---|
| 104*j | 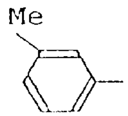 (S-form) | H | H | H | H | 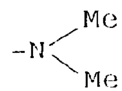 | 1 | 1 | HCl | 163-164.5 [EtOH-$Me_2CO$] |
| 105*k | 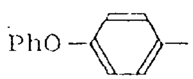 (S-form) | " | " | " | " | " | " | " | " | 187.5-188.5 [MeCN] |
| 106*h | 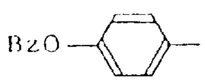 | " | " | " | " | 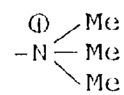 | " | " | 1/2 1,5-Naphthalenedisulfonic acid | 168-170 (decomp.) |
| 107 | 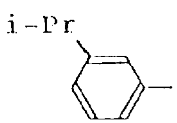 | " | " | " | " | 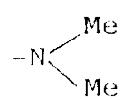 | " | " | - | Oily |

Table 5 (cont'd)

| | | |
|---|---|---|
| 108 | 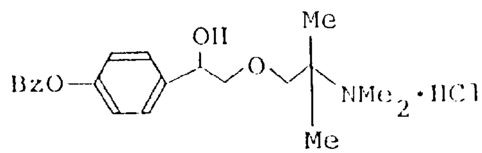 | 171-173 [EtOH-$Et_2O$] |

Note: *a: Optical rotation: -43.3° (27°C, C=3, EtOH)

*b: Optical rotation: +43.3° (24°C, C=3, EtOH)

*c: Obtained by treating compound No. 81 with p-toluenesulfonic acid.

*d: A trityl group was used as an amino-protecting group.

*e: Optical rotation: -38.9° (28°C, C=1.5, MeOH)

*f: Optical rotation: -45.8° (25°C, C=1.5, MeOH)

*g: Optical rotation: -38.5° (25°C, C=1.5, EtOH)

*h: Obtained by reacting 1 mole of a dimethylamino form with 0.5 mole of dimethyl 1,5-naphthalenedisulfonate.

*i: Optical rotation: +37.3° (23°C, C=1.5, MeOH)

*j: Optical rotation: +40.2° (23°C, C=1.5, MeOH)

*k: Optical rotation: +28.4° (23°C, C=1.5, EtOH)

Production Example 4

(1) 5.1 g of potassium tert-butoxide was added to 105 ml of ethylene glycol mono-tert-butyl ether. The mixture was heated to 80°C. Thereto was dropwise added 35.0 g of 2-(3-chlorophenyl)oxirane over 1 hour. The mixture was stirred for 2 hours at the same temperature. The reaction mixture was added to a mixture of 100 ml of ice water and 100 ml of ethyl acetate. The organic layer was separated, washed with a saturated aqueous sodium chloride solution, and dried over anhydrous magnesium sulfate. The solvent was removed by distillation under reduced pressure. The residue thus obtained was subjected to further distillation under reduced pressure to obtain 37.7 g of colorless oily 1-(3-chlorophenyl) -2-(2-tert-butoxyethoxy)ethanol having a boiling point of 140-146°C/120 Pa (0.9 mmHg).
(2) 37.0 g of 1-(3-chlorophenyl)-2-(2-tert-butoxyethoxy)ethanol was dissolved in 70 ml of methylene chloride. To the solution were added 12.9 g of pyridine and 16.6 g of acetic anhydride. The resulting mixture was stirred for 24 hours at room temperature. The reaction mixture was added to a mixture of 150 ml of ice water and 100 ml of methylene chloride. The resulting mixture was adjusted to pH 2 with 6 N hydrochloric acid. The organic layer was separated, washed with a saturatred aqueous sodium hydrogencarbonate solution and water in this order, and dried over anhydrous magnesium sulfate. The solvent was removed by distillation under reduced pressure to obtain 40.0 g of oily 1-acetoxy-1-(3-chlorophenyl)-2-(2-tert-butoxyethoxy)ethane.
(3) 40.0 g of 1-acetoxy-1-(3-chlorophenyl)-2-(2-tert-butoxyethoxy)ethane was dissolved in 40 ml of methylene chloride. To the solution was added 80 ml of trifluoroacetic acid with ice cooling. The mixture was stirred for 12 hours at room temperature. After the completion of the reaction, the solvent was removed by distillation under reduced pressure. The residue was mixed with 100 ml of toluene. The solvent was further removed by distillation under reduced pressure. The residue thus obtained was dissolved in a mixture of 90 ml of ethanol and 10 ml of water. To the solution was added 10.7 g of sodium hydrogencarbonate at room temperature. The mixture was adjusted to pH 6-7 with a saturated aqueous sodium hydrogencarbonate solution, and then concentrated to about a half volume under reduced pressure. To the concentrate was added 100 ml of ethyl acetate. The organic layer was separated. The aqueous layer was extracted with 50 ml of ethyl acetate. The extract was combined with the previously separated organic layer. The combined organic layer was washed with a saturated aqueous sodium chloride solution and dried over anhydrous magnesium sulfate. The solvent was removed by distillation under reduced pressure. The residue was purified by column chromatography (eluant: toluene/ethyl acetate = 3/l) to obtain 19.4 g of oily 1-acetoxy-1-(3-chlorophenyl)-2-(2-hydroxyethoxy)-ethane.
(4) To a mixture of 19.0 g of 1-acetoxy-1-(3-chlorophenyl)-2-(2-hydroxyethoxy)ethane and 95.0 ml of methylene chloride containing 9.1 ml of methanesulfonyl chloride was dropwise added 16.4 ml of triethylamine with ice cooling over 1 hour. The resulting mixture was stirred for 10 minutes at the same temperature and further for 1 hour at room temperature. The reaction mixture was added to a mixture of 50.0 ml of methylene chloride and 50.0 ml of ice water. The resulting mixture was adjusted to pH 2.0 with 6 N hydrochloric acid. The organic layer was separated, washed with water and dried over anhydrous magnesium sulfate. The solvent was removed by distillation under reduced pressure to obtain 24.5. g of oily 1-acetoxy-1-(3-chlorophenyl)-2-(2-methanesulfonyloxyethoxy)ethane.
(5) 1.40 g of 1-acetoxy-1-(3-chlorophenyl)-2-(2-methanesulfonyloxyethoxy)ethane was dissolved in 7 ml of N,N-dimethylformamide. To the solution were added 0.69 ml of N-methylpiperazine and 1.03 g of potassium carbonate. The mixture was stirred for 3 hours at 80°C. The reaction mixture was cooled and added to a mixture of 30 ml of ice water and 30 ml of diethyl ether. The organic layer was separated. The aqueous layer was extracted with 20 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was mixed with 7 ml of methanol and 45 mg of sodium methoxide. The mixture was allowed to stand overnight at room temperature. The solvent was removed by distillation under reduced pressure. To the residue thus obtained were added 20 ml of ethyl acetate and 10 ml of water. The organic layer was separated. The aqueous layer was extracted with 20 ml of ethyl acetate. The extract was combined with the previously separated organic layer. The combined organic layer was washed with a saturated aqueous sodium chloride solution and then purified by column chromatography (eluant: chloroform/methanol = 10/l). To the resulting oily product were added 10 ml of ethanol and 1.2 ml of a 6 N dry hydrogen chloride-ethanol solution in this order. To the resulting mixture was added 10 ml of diethyl ether. The mixture was stirred for 30 minutes. The resulting crystals were collected by filtration, washed with a mixture of 2 ml of ethanol and 2 ml of diethyl ether, and dried to obtain 770 mg of 1-(3-chlorophenyl)-2-[2-(4-methylpiperazin-1-yl)ethoxy]ethanol dihydrochloride (compound No. 109).

Melting point: 211-213°C [MeOH-EtOH]
The compounds shown in Table 6 were obtained in the same manner.
In Table 6, $R^1$, $R^2$, $R^3$, $R^4$, $R^6$ and n each show a substituent or integer used in the following formula:

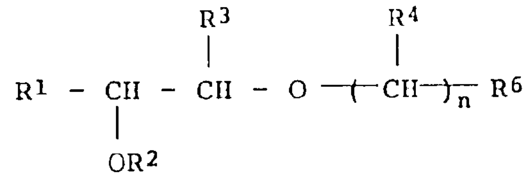

Table 6

| Compound No. | $R^1$ | $R^2$ | $R^3$ | $R^4$ | $R^6$ | n | Addition salt | Melting point (°C) |
|---|---|---|---|---|---|---|---|---|
| 110 | 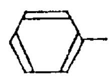 | H | H | H | 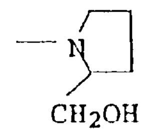 | 2 | - | Oily |
| 111 | " | " | " | " | 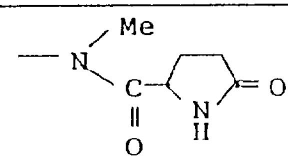 | " | " | 134.5-136.5 [IPA] |
| 112 | " | " | " | " | 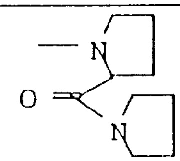 | " | " | Oily |
| 113 | " | " | " | " | 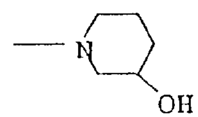 | " | HCl | 138-140 [IPA] |

- Cont'd -

Table 6 (Cont'd)

| | | | | | | | | |
|---|---|---|---|---|---|---|---|---|
| 114 | 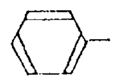 | H | H | H | 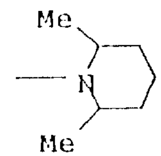 | 2 | - | Oily |
| 115 | " | " | " | " | 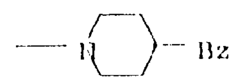 | " | HCl | 164.5 - 166.5 |
| 116 | " | " | " | " | 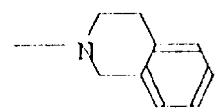 | " | " | 169-171 [EtOH] |
| 117 | " | " | " | " | 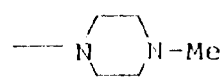 | " | 2HCl | 169-171 [$CH_2Cl_2$-EtOH] |
| 118 | " | " | " | " | 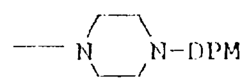 | " | - | 104.5 - 105.5 [EtOH] |

- Cont'd -

Table 6 (Cont'd)

| | | | | | | | | |
|---|---|---|---|---|---|---|---|---|
| 119 | 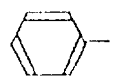 | H | H | H | 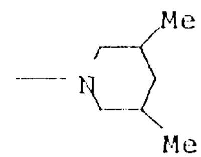 | 2 | - | Oily |
| 120[*a] | " | " | " | " | 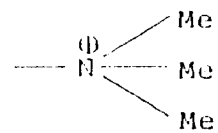 | " | 1/2 1,5-Naphthalenedisulfonic acid | 208-210 [MeOH-$Et_2O$] |

Note: *a: 1 mole of a dimethylamino form was reacted with 0.5 mole of dimethyl 1,5-naphthalenedisulfonate, to obtain the desired product.

Production Example 5

A mixture of 2.0 g of 1-(4-benzyloxyphenyl)-2-[2-(N,N-dimethylamino)ethoxy]ethanol hydrochloride, 500 mg of 10% palladium-carbon and 10 ml of methanol was subjected to hydrogenation for 2 hours at room temperature under atmospheric pressure. After the completion of the reaction, palladium-carbon was removed by filtration. The solvent was removed by distillation under reduced pressure to obtain 1.4 g of 1-(4-hydroxyphenyl)-2-[2-(N,N-dimethylamino)ethoxy]ethanol hydrochloride (Compound No. 121)

Melting point: 169.5-170.5°C [EtOH]

The compounds shown in Table 7 were obtained in the same manner.

In Table 7 , $R^1$, $R^2$, $R^3$, $R^4$, $R^6$ and n each show a substituent or integer used in the following formula:

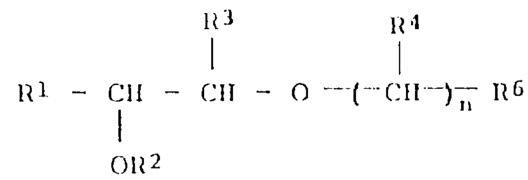

Table 7

| Compound No. | R1 | R2 | R3 | R4 | R6 | n | Addition salt | Melting point (°C) |
|---|---|---|---|---|---|---|---|---|
| 122 | 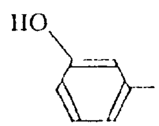 | H | H | H | 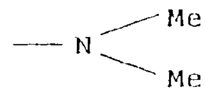 | 2 | HCl | 150-152 [EtOH] |
| 123 | " | Ac | " | " | " | " | " | Amorphous |
| 124 | 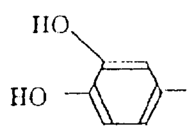 | H | " | " | " | " | - | Oily |
| 125 | 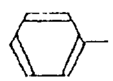 | " | " | " | 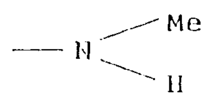 | " | HCl | 145-147 |

- Cont'd -

Table 7 (Cont'd)

| | | | | | | | | |
|---|---|---|---|---|---|---|---|---|
| 126 | 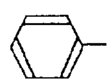 | H | H | H | 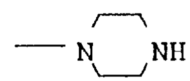 | 2 | 2HCl | 204.5 - 206.5 [MeOH] |
| 127 | 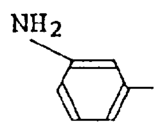 | " | " | " | 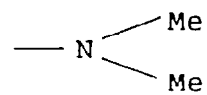 | " | HCl | 151-153 |

Production Example 6

0.65 ml of pyridine and 0.99 ml of acetic anhydride were added to 1 g of 1-(3-methylphenyl)-2-[2-(morpholin-4-yl) ethoxy]ethanol. The mixture was stirred for 3 hours at room temperature. The reaction mixture was subjected to distillation under reduced pressure to remove the solvent. To the residue were added 20 ml of ethyl acetate and 10 ml of water. The mixture was adjusted to pH 10 with potassium carbonate. The organic layer was separated, washed with water and a saturated aqueous sodium chloride solution in this order, and dried over anhydrous magnesium sulfate. The solvent was removed by distillation under reduced pressure. The residue was purified by column chromatography (eluant: chloroform/ethanol = 30/l) to obtain 1 g of oily 1-acetoxy-1-(3-methylphenyl)-2-[2-(morpholin-4-yl)ethoxy] ethane (compound No. 128).

The compounds shown in Table 8 were obtained in the same manner.

In Table 8 , $R^1$, $R^2$, $R^3$, $R^4$, $R^6$ and n each show a substituent or integer used in the following formula:

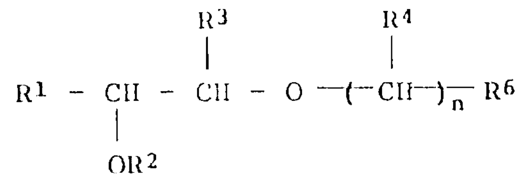

Table 8

| Compound No. | $R^1$ | $R^2$ | $R^3$ | $R^4$ | $R^6$ | n | Addition salt | Melting point (°C) |
|---|---|---|---|---|---|---|---|---|
| 129 | 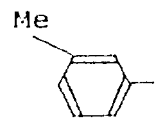 | Ac | H | H | 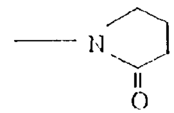 | 2 | – | Oily |
| 130 | " | " | " | " | 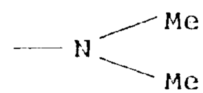 | " | " | " |
| 131*d | 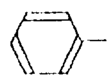 | H | " | " | 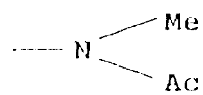 | " | " | " |
| 132*e | 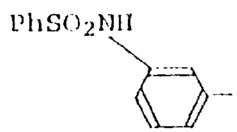 | " | " | " | 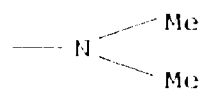 | " | HCl | 174.5 – 176.5 [EtOH] |

- Cont'd -

Table 8 (Cont'd)

| | | | | | | | | |
|---|---|---|---|---|---|---|---|---|
| 133 | 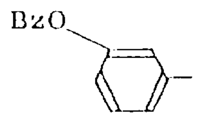 | Ac | H | H | 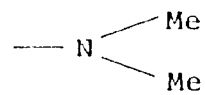 | 2 | HCl | 169.5 - 170.5 [EtOH] |
| 134 | 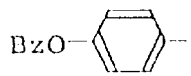 | " | " | " | " | " | " | 131.5 - 132.5 [$Me_2CO$] |
| 135 | 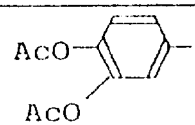 | " | " | " | " | " | " | Oily |
| 136 | 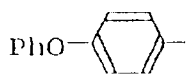 | " | " | " | " | " | – | Oily |
| 137 | 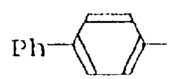 | " | " | " | " | " | " | 144.5 - 146.5 [EtOH-$Et_2O$] |

- Cont'd -

Note: *d: The indicated compound was obtained by reacting compound No. 125 with acetic anhydride.

*e: The indicated compound was obtained by reacting compound No. 127 with benzenesulfonyl chloride in pyridine.

Production Example 7

50 ml of a 1:1 mixture of chloroform and water was added to 3.30 g of 1-acetoxy-1-(3-hydroxyphenyl)-2-[2-(N,N-dimethylamino)ethoxy]ethane hydrochloride. To the mixture was added 750 mg of sodium carbonate with ice cooling. The organic layer was separated and dried with anhydrous magnesium sulfate. The solvent was removed by distillation under reduced pressure. The residue was mixed with 20 ml of benzene. The mixture was heated to 60°C. To the resulting solution was dropwise added a solution of 870 mg of ethyl isocyanate dissolved in 5 ml of benzene in 10 minutes. The mixture was stirred for 40 minutes at the same temperature. After the completion of the reaction, the solvent was removed by distillation under reduced pressure. The residue thus obtained was purified by column chromatography (eluant: chloroform/ethanol = 10/l) to obtain an oily product. The oily product was dissolved in 10 ml of ethanol. Hydrogen chloride gas was blown into the solution. The solvent was removed by distillation under reduced pressure to obtain 1.90 g of 1-acetoxy-1-[3-(N-ethylcarbamoyloxy)phenyl]-2-[2-(N,N-dimethylamino)-ethoxy]ethane hydrochloride (compound No. 138).

Melting point: 111.5-113.5°C [$Me_2CO$]

In the same manner, there was obtained oily 1-acetoxy-1-[3-(3-chlorophenylcarbamoyloxy)phenyl]-2-[2-(N,N-dimethylamino)ethoxy]ethane hydrochloride (compound No. 139).

Production Example 8

(1) 9.5 g of potassium tert-butoxide was added to 92 ml of ethylene glycol. The mixture was heated to 80°C. Thereto was dropwise added, in 3 hours, a solution of 34.7 g of 2-(4-benzyloxyphenyl)oxirane dissolved in 220 ml of dimethyl sulfoxide. The resulting mixture was stirred for 30 minutes at the same temperature. The reaction mixture was cooled and added to a mixture of 1 liter of ice water and 600 ml of ethyl acetate. The resulting mixture was adjusted to pH 7 with 6 N hydrochloric acid. The organic layer was separated. The aqueous layer was extracted with 200 ml of ethyl acetate. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 10/l) to obtain 25 g of 1-(4-benzyloxyphenyl)-2-(2-hydroxyethoxy)ethanol.

Melting point: 116.5-117°C [MeCN]

(2) 22.7 g of 1-(4-benzyloxyphenyl)-2-(2-hydroxyethoxy)ethanol was dissolved in 140 ml of pyridine. The solution *was* cooled to -20°C. To the solution was added 19 g of p-toluenesulfonyl chloride. The resulting mixture was heated to 0°C and stirred for 15 hours at the same temperature. The rection mixture was added to a mixture of 300 ml of ice water and 200 ml of diethyl ether. The resulting mixture was adjusted to pH 2 with 6 N hydrochloric acid. The organic layer was separated. The aqueous layer was extracted with 100 ml of diethyl ether. The extract was combined with the previously separated organic layer. The combined organic layer was washed with 1 N hydrochloric acid, water and a saturated aqueous sodium chloride solution in this order, and dried over anhydrous magnesium sulfate. The solvent was removed by distillation under reduced pressure. The residue was purified by column chromatography (eluant: toluene/ethyl acetate = 3/l) to obtain 20.5 g of oily 1-(4-benzyloxyphenyl)-2-[2-(p-toluenesulfonyl)ethoxy]ethanol.

(3) To a mixture of 20 g of 1-(4-benzyloxyphenyl)-2-[2-(p-toluenesulfonyloxy)ethoxy]ethanol and 40 ml of methylene chloride containing 8.2 g of 3,4-dihydro-2H-pyrane was added 2.3 g of pyridinium p-toluenesulfonate at room temperature. The resulting mixture was refluxed for 30 minutes. The reaction mixture was cooled and added to a mixture of 100 ml of ice water and 100 ml of methylene chloride. The organic layer was separated, washed with water, and dried over anhydrous magnesium sulfate. The solvent was removed by distillation under reduced pressure to obtain 18.6 g of light yellow oily 1-(4-benzyloxyphenyl)-1-(2-tetrahydropyranyloxy)-2-[2-(p-toluenesulfonyloxy)ethoxy]ethane.

(4) A mixture of 2 g of 1-(4-benzyloxyphenyl)-1-(2-tetrahydropyranyloxy)-2-[2-(p-toluenesulfonyloxy)ethoxy] ethane, 5.9 ml of a 40% aqueous methylamine solution and 20 ml of ethanol was refluxed for 2 hours. The reaction mixture was cooled and added to a mixture of 50 ml of ice water and 50 ml of diethyl ether. The organic layer was separated. The aqueuos layer was extracted twice each with 20 ml of diethyl ether. The extracts were combined with the previously separated organic layer. The combined organic layer was mixed with 10 ml of water. The mixture was adjusted to pH 1.0 with 6 N hydrochloric acid. The aqueous layer was separated and stirred for 30 minutes at room temperature. Thereto was added 30 ml of chloroform. The resulting mixture was adjusted to pH 12 with a 5% aqueous sodium hydroxide solution. The organic layer was separated, washed with water and a saturated aqueous sodium chloride solution in this order, and dried over anhydrous magnesium sulfate. The solvent was removed by distillation under reduced pressure. The residue was mixed with 10 ml of acetone. Hydrogen chloride gas was blown into the mixture with ice cooling. The resulting crystals were collected by filtration, washed with

acetone, and dried to obtain 620 mg of 1-(4-benzyloxyphenyl)-2-(2-methylaminoethoxy)ethanol hydrochloride (compound No. 140).

Melting point: 173-173.5°C [EtOH]

The compounds shown in Table 9 were obtained in the same manner.

In Table 9 , $R^1$, $R^2$, $R^3$, $R^{4a}$, $R^{4b}$, $R^6$, na and nb each show a substituent or integer used in the following formula:

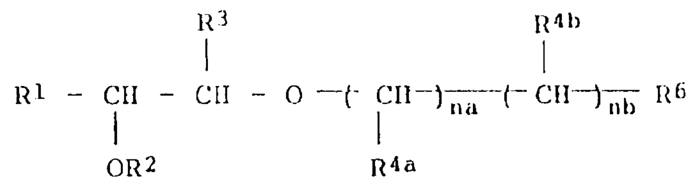

Table 9

| Compound No. | $R^1$ | $R^2$ | $R^3$ | $R^{4a}$ | $R^{4b}$ | $R^6$ | na | nb | Addition salt | Melting point (°C) |
|---|---|---|---|---|---|---|---|---|---|---|
| 141 | 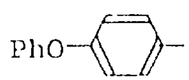 | H | H | H | H | -NMe<br>H | 1 | 1 | HCl | 158-158.5 [IPA] |
| 142 | " | " | " | " | " | -N-i-Pr<br>H | " | " | " | 170-170.5 [EtOH] |
| 143 | 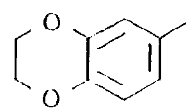 | " | " | " | " | 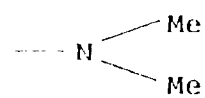 | " | " | " | 180-181 [EtOH-AcOEt] |
| 144 | 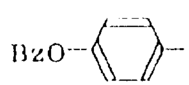 | " | " | " | " | 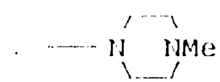 | " | " | 2HCl | 178-180.5 [MeCN-$Et_2O$] |

- Cont'd -

Table 9 (Cont'd)

| | | | | | | | | | | |
|---|---|---|---|---|---|---|---|---|---|---|
| 145 | 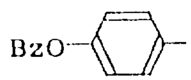 | H | H | H | H | -N-i-Pr<br>H | 1 | 1 | HCl | 174.5-175 [IPA] |
| 146 | " | " | " | " | " | 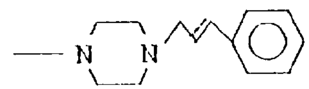 | " | " | 2HCl | 212.5-213 [MeOH] |
| 147 | " | " | " | " | " | 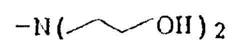 | " | " | – | Oily |
| 148 | " | " | " | " | " | 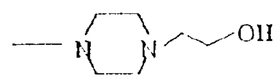 | " | " | 2HCl | 158.5 – 160.5 [EtOH] |
| 149 | " | " | " | " | " | 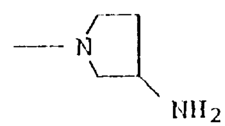 | " | " | – | 55-58 |

– Cont'd –

Table 9 (Cont'd)

| | | | | | | | | | | |
|---|---|---|---|---|---|---|---|---|---|---|
| 150 | 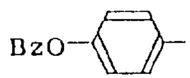 | H | H | H | H | 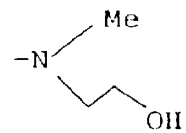 | 1 | 1 | - | 36-38 |
| 151 | " | " | " | " | " | 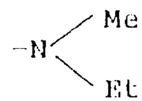 | " | " | " | Oily |
| 152 | " | " | " | " | " | 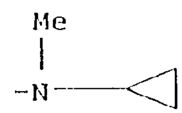 | " | " | HCl | 139-141 [IPA-AcOEt] |
| 153 | " | " | " | " | " | 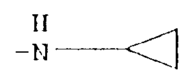 | " | " | " | 163-164 [EtOH-MeCN] |
| 154 | " | " | " | " | " | 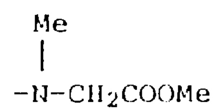 | " | " | - | Oily |

- Cont'd -

Table 9 (Cont'd)

| | | | | | | | | | | |
|---|---|---|---|---|---|---|---|---|---|---|
| 155 | 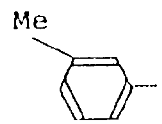 | H | H | H | H | 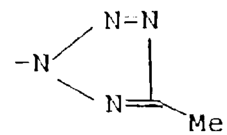 | 1 | 1 | - | Oily |
| 156 | " | " | " | " | " | 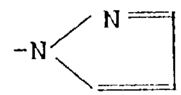 | " | " | " | " |
| 157 | " | " | " | " | " | 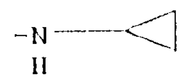 | " | " | HCl | 148.5 - 149.5 [IPA-AcOEt] |
| 158 | 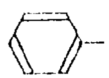 | " | " | " | " | " | " | " | " | 173.5 - 175.5 [EtOH-$Me_2CO$] |
| 159 | " | " | " | " | " | 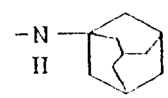 | " | " | PTS | 198-200 [EtOH-AcOEt] |

- Cont'd -

Table 9 (Cont'd)

| | | | | | | | | | | |
|---|---|---|---|---|---|---|---|---|---|---|
| 160 | 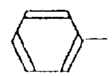 | H | H | H | H | -N-t-Bu<br>H | 1 | 1 | HCl | 161-162.5<br>[IPA-<br>AcOEt] |
| 161[*1] | 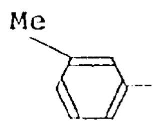 | Me | " | " | " | 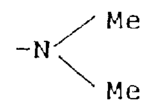 | " | " | " | Oily |
| 162[*1] | 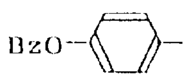 | " | " | " | " | " | " | " | – | " |

Note: *1: Methoxylation was effected using diazomethane-boron trifluoride in place of using 3,4-dihydro-2H-pyran, to obtain the desired product.

Production Example 9

1.23 g of N,N'-dicyclohexylcarbodiimide was added, with ice cooling, to a solution of 1.08 g of 2-(2-aminoethoxy)-1-phenylethanol, 730 mg of nicotinic acid, 810 mg of 1-hydroxybenzotriazole and 0.83 ml of triethylamine dissolved in 5 ml of tetrahydrofuran. The resulting mixture was stirred at the same temperature for 5 minutes and further at room temperature for 1 hour. To the reaction mixture was added 11 ml of ethyl acetate. The insolubles were removed by filtration. To the filtrate was added 5 ml of water and the mixture was adjusted to pH 2 with 6 N hydrochloric acid. The aqueous layer was separated. The organic layer was extracted with 5 ml of water. The extract was combined with the previously separated aqueous layer. The combined aqueous layer was mixed with 20 ml of chloroform and adjusted to pH 10 with potassium carbonate. The organic layer was separated, washed with water, and dried over anhydrous magnesium sulfate. The solvent was removed by distillation under reduced pressure. The residue thus obtained was purified by column chromatography (eluant: chloroform/ethanol = 15/l) to obtain 400 mg of oily 2-(2-nicotinoylaminoethoxy)-1-phenylethanol (compound No. 163).

IR (neat) $cm^{-1}$: $\nu_{C=O}$ 1635

Production Example 10

(1) 1,19 g of 4-benzylthiobenzaldehyde was dissolved in 20 ml of tetrahydrofuran. The solution was cooled to -10°C. Thereto was dropwise added 10 ml of a tetrahydrofuran solution containing 2 M of 2-chloroethoxymethylmagnesium chloride in 10 minutes. The resulting mixture was stirred for 1 hour with ice cooling. The reaction mixture was added to a mixture of 50 ml of ice water, 50 ml of ethyl acetate and 1 g of ammonium chloride. The resulting mixture was adjusted to pH 2 with 6 N hydrochloric acid. The organic layer was separated, washed with water and a saturated aqueous sodium chloride solution in this order, and dried over anhydrous magnesium sulfate. The solvent was removed by distillation under reduced pressure. The residue thus obtained was purified by column chromatography [eluant: toluene/ethyl acetate = 20/l] to obtain 1.34 g of 1-(4-benzylthiophenyl)-2-(2-chloroethoxy) ethanol.

Melting point: 49.5-50.5°C [hexane-IPE]

(2) A mixture of 600 mg of 1-(4-benzylthiophenyl)-2-(2-chloroethoxy)ethanol, 4 ml of a 50% aqueous dimethylamine solution, 310 mg of potassium iodide and 5 ml of ethanol was refluxed for 4 hours. To the reaction mixture was further added 4 ml of a 50% aqueous dimethylamine solution. The resulting mixture was refluxed for 4 hours. The reaction mixture was cooled to room temperature. The solvent was removed by distillation under reduced pressure. To the residue thus obtained were added 30 ml of ethyl acetate and 30 ml of water. The resulting mixture was adjusted to pH 10.5 with potassium carbonate. The organic layer was separated, washed with water and a saturated aqueous sodium chloride solution in this order, and dried over anhydrous magnesium sulfate. The solvent was removed by distillation under reduced pressure. The residue was dissolved in 15 ml of acetone. To the solution was added 0.4 ml of a 5 N dry hydrogen chloride-ethanol solution. The resulting crystals were collected by filtration to obtain 590 mg of 1-(4-benzylthiophenyl)-2-[2-(N,N-dimethylamino)ethoxy]ethanol hydrochloride (compound No. 164).

Melting point: 173.5-174.5°C [EtOH]

The compounds shown in Table 10 were obtained in the same manner.

In Table 10, $R^1$, $R^2$, $R^3$, $R^{4a}$, $R^{4b}$, $R^6$, na and nb each show a substitutent or integer used in the following formula:

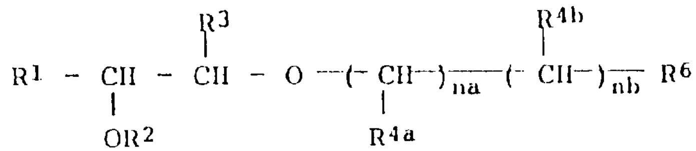

Table 10

| Compound No. | R1 | R2 | R3 | R4a | R4b | R6 | na | nb | Addition salt | Melting point (°C) |
|---|---|---|---|---|---|---|---|---|---|---|
| 165 | 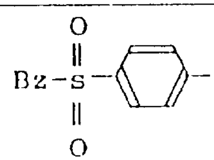 | H | H | H | H | 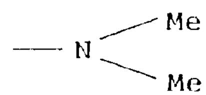 | 1 | 1 | HCl | 194-195 [EtOH] |
| 166 | 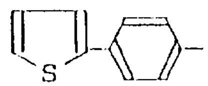 | " | " | " | " | " | " | " | " | 207-208 [EtOH-AcOEt] |
| 167 | 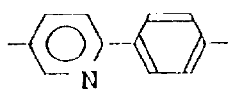 | " | " | " | " | " | " | " | Maleic acid | 168-170 (decomp.) |
| 168 | 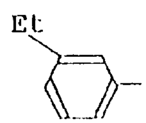 | " | " | " | " | " | " | " | HCl | 159.5 - 160.5 [EtOH-AcOEt] |
| 169 | 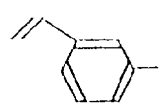 | " | " | " | " | " | " | " | " | 141-142 [EtOH-AcOEt] |

Next, this invention is specifically described by way of Examples. However, this invention is in no way restricted to these Examples.

Example 1 (Tablets)

Tablets each containing 50 mg of 1-(4-benzyloxyphenyl)-2-[2-(N,N-dimethylamino)ethoxy]ethanol hydrochloride (compound No. 48) were prepared using the following recipe according to the following method:

| Per tablet: | | |
|---|---|---|
| Compound No. 48 | 50 mg | ① |
| Lactose | 20 mg | ① |
| Kollidon CL (BASF's product) | 15 mg | ① |
| Corn starch | 30 mg | ① |
| AVICEL PH 101 (Asahi Kasei's product) | 50 mg | ① |
| Polyvinylpyrrolidone K-90 | 5 mg | |
| Light silica | 3 mg | ② |
| Magnesium stearate | 2 mg | ② |
| Total | 175 mg | |

The components ① were kneaded with an aqueous solution containing 8% of Polyvinylpyrrolidone K-90. The kneaded product was dried at 40°C and mixed with the components ②. The resulting mixture was made into round tablets each weighing 175 mg and having a diameter of 8 mm.

Example 2 (Capsules)

Capsules each containing 50 mg of 2-[2-(N,N-dimethylamino)ethoxy]-1-[4-(4-phenyloxy)phenyl]ethanol hydrochloride (compound No. 54) were prepared using the following recipe according to the following method:

| Per capsule: | | |
|---|---|---|
| Compound No. 54 | 50 mg | ① |
| Lactose | 20 mg | ① |
| Corn starch | 53 mg | ① |
| Kollidon CL (BASF's product) | 2 mg | ① |
| Polyvinylpyrrolidone K-90 | 5 mg | |
| AVICEL PH 302 (Asahi Kasei's product) | 18 mg | ② |
| Magnesium stearate | 2 mg | ② |
| Total | 150 mg | |

The components① were kneaded with an aqueous solution containing 8% of Polyvinylpyrrolidone K-90. The kneaded product was dried at 40°C and mixed with the components②. The resulting mixture was charged into No. 3 gelatin capsules in an amount of 150 mg per capsule to obtain capsules.

Example 3 (Liquid)

A liquid containing 25 mg of 2-[2-(N,N-dimethylamino)ethoxy]-1-(3-trifluoromethylphenyl)ethanol hydrochloride (compound No. 42) was prepared using the following recipe according to the following method:

| Per ampule: | |
|---|---|
| Compound No. 42 | 25 mg |
| Methyl paraoxybenzoate | 1 mg |
| Total | 26 mg |

The above components were dissolved in physiological saline and the total volume of the solution was made into 1 ml. The solution was filtered aseptically and charged into an ampule to obtain a liquid.

Example 4 (Injection)

An injection containing 25 mg of 2-[2-(N,N-dimethylamino)ethoxy]-1-(3-fluorophenyl)ethanol hydrochloride (compound No. 1) was prepared using the following recipe according to the following method:

| | |
|---|---|
| Compound No. 1 | 25 mg |
| Manitol | 75 mg |
| Total | 100 mg |

The above components were dissolved in 1.5 ml of distilled water prepared for injection. The solution was filtered aseptically, charged into a 3-ml minivial, and freeze-dried to obtain an injection.

Example 5 (fine granules)

Fine granules each containing 50 mg of 2-(1-benzylpiperidin-4-yloxy)-1-phenylethanol hydrochloride (compound No. 108) were prepared using the following recipe according to the following method:

| | | |
|---|---|---|
| Compound No. 108 | 50 mg | ① |
| α-Starch | 200 mg | ① |
| Purified sucrose | 250 mg | ① |
| Lactose | 470 mg | ① |
| Polyvinylpyrrolidone K-90 | 30 mg | |
| Total | 1000 mg | |

The components ① were subjected to granulation under high speed stirring with an aqueous solution containing 8% of Polyvinylpyrrolidone K-90. The granules obtained were sieved through a 32-mesh screen and dried to obtain fine granules.

Example 6 (Tablets)

2-[2-(N,N-dimethylamino)ethoxy]-1-(3-methylphenyl)ethanol hydrochloride (compound No. 15) was processed as in Example 1 to obtain tablets containing 50 mg of the compound.

Example 7 (Capsules)

2-[2-(N,N-dimethylamino)ethoxy]-1-(3-methyl-phenyl)ethanol hydrochloride (compound No. 15) was processed as in Example 2 to obtain capsule containing 50 mg of the compounds.

## Claims

1. A 1,2-ethanediol derivative represented by the following general formula or a salt thereof:

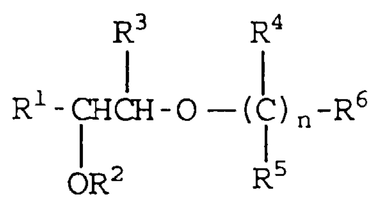

wherein $R^1$ represents a substituted or unsubstituted phenyl group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; $R^6$ represents a substituted or unsubstituted amino or nitrogen-containing heterocyclic group, the nitrogen-containing heterocyclic group having a free valence on a nitrogen atom forming the heterocyclic ring and being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6, wherein the substituent on $R^1$ is selected from the group consisting of halogen atoms, substituted or unsubstituted amino, $C_{1-6}$ alkyl, aryl , ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$

alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected hydroxyl groups, protected or unprotected amino groups, unsubstituted $C_{1-6}$ alkyl groups, $C_{1-6}$ alkyl groups substituted by a protected or unprotected hydroxyl group, halogen-substituted aryl groups, halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups , ar-$C_{1-4}$ alkenyl groups, heterocyclic groups, oxo group, $C_{1-6}$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, unsubstituted or oxo-substituted heterocyclic-CO-groups, adamantyl group, $C_{1-6}$ alkylsulfonyl groups and arylsulfonyl groups, provided that there are excluded the compounds in which $R^1$ represents an unsubstituted or $C_{1-6}$ alkyl-substituted phenyl group and $R^6$ represents a di-$C_{1-6}$ alkylamino group,

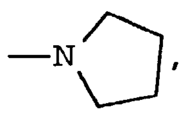 , 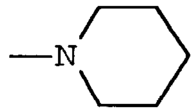 or 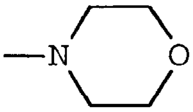 ;

all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo [2,3-a]pyridyl, benzo [b] piperazinyl, chromenyl, isothiazolyl, isoxazolyl, oxadiazolyl, pyridazinyl, isoindolyl and isoquinolyl groups.

2. A 1,2-ethanediol derivative or a salt thereof according to Claim 1, wherein $R^2$ represents a hydrogen atom or a hydroxyl-protecting group; $nR^{4}$'s are the same as or different from one another and represent hydrogen atoms or $C_{1-6}$ alkyl groups; $nR^{5}$'s represent hydrogen atoms; and $R^6$ represents a substituted or unsubstituted amino (other than di-$C_{1-6}$ alkylamino) or nitrogen-containing heterocyclic group, the nitrogen-containing heterocyclic group having a free valence on a nitrogen atom forming the heterocyclic ring and being selected from the group consisting of pyrrolyl, piperazinyl, imidazolyl, pyrazolyl, pyridyl, pyrimidinyl, quinolyl, quinolizinyl, tetrahydroquinolinyl, quinuclidinyl, thiazolyl and thiadiazolyl groups.

3. 1-(4-Benzyloxyphenyl)-2-[2-(N,N-dimethylamino)ethoxy]ethanol or a salt thereof.

4. A process for producing a 1,2-ethanediol derivative represented by the following general formula or a salt thereof:

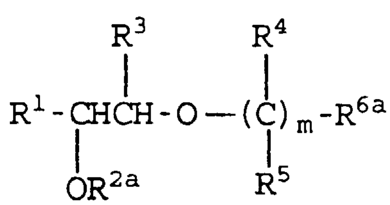

wherein $R^1$ represents a substituted or unsubstituted phenyl group; $R^{2a}$ represents a hydroxyl-protecting group; $R^3$, $R^4$, $R^5$ and all the heterocyclic groups are as defined in Claim 1, and m is the same as n defined in Claim 1 and $R^{6a}$ is the same as $R^6$ defined in Claim 1, which comprises reacting a compound represented by the general formula:

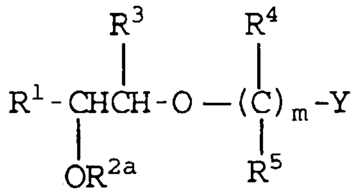

wherein $R^1$, $R^{2a}$, $R^3$, $R^4$, $R^5$ and m have the same meanings as defined above and Y represents a removable group, with a compound represented by the following general formula or a salt thereof:

$$H\text{-}R^{6a}$$

wherein $R^{6a}$ has the same meaning as defined above.

**5.** Use of a 1,2-ethanediol derivative represented by the following general formula or a salt thereof for preparing a drug for treating cerebrovascular dementia, senile dementia, Alzheimer's dementia, sequelae of ischemic encephalopathy or cerebral apoplexy in a patient:

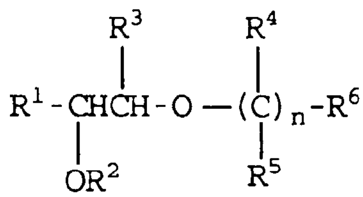

wherein $R^1$ represents a substituted or unsubstituted phenyl group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; $R^6$ represents an ammonio group or a substituted or unsubstituted amino or nitrogen-containing heterocyclic group, the nitrogen-containing heterocyclic group having a free valence on a nitrogen atom forming the heterocyclic ring and being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$-alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected hydroxyl groups, protected or unprotected amino groups, unsubstituted $C_{1-6}$ alkyl groups, $C_{1-6}$ alkyl groups substituted by a protected or unprotected hydroxyl group, halogen-substituted aryl groups, halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{2-6}$ alkenyl groups, heterocyclic groups, oxo group, $C_{1-6}$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, unsubstituted or oxo-substituted heterocyclic-CO-groups, adamantyl group, $C_{1-6}$ alkylsulfonyl groups and arylsulfonyl groups; all the above heterocyclic groups being selected from the group consisting

of the nitrogen-containing heterocyclic groups mentioned in the definition of $R^6$ and furyl, thienyl, benzothienyl pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo [2,3-a] pyridyl, benzo [b] piperazinyl, chromenyl, isothiazolyl, isoxazolyl, oxadiazolyl, pyridazinyl, isoindolyl and isoquinolyl groups.

**6.** Use of a 1,2-ethanediol derivative or a salt thereof according to Claim 5, wherein $R^2$ represents a hydrogen atom or a hydroxyl-protecting group; $nR^4$'s are the same as or different from one another and represent hydrogen atoms or $C_{1-6}$ alkyl groups; $nR^5$'s represent hydrogen atoms; and $R^6$ represents an ammonio group or a substituted or unsubstituted amino or nitrogen-containing heterocyclic group, the nitrogen-containing heterocyclic group having a free valence on a nitrogen atom forming the heterocyclic ring and being selected from the group consisting of pyrrolyl, pyrrolidinyl, piperidinyl, piperazinyl, imidazolyl, pyrazolyl, pyridyl, pyrimidinyl, morpholinyl, quinolyl, quinolizinyl, tetrahydroquinolinyl, quinuclidinyl, thiazolyl and thiadiazolyl groups.

**7.** Use of a 1,2-ethanediol derivative or a salt thereof according to Claim 5, wherein $R^6$ represents an ammonio group or a substituted or unsubstituted amino or nitrogen-containing heterocyclic group, the nitrogen-containing heterocyclic group having a free valence on a nitrogen atom forming the heterocyclic ring and being selected from the group consisting of pyrrolinyl, piperidyl, piperazinyl, imidazolyl, pyridyl, morpholinyl, thiomorpholinyl, tetrahydropyridyl, quinuclidinyl and tetrazolyl, the substituent on $R^1$ is selected from the group consisting of halogen atoms; a $C_{1-6}$ alkyl group; $C_{2-6}$ alkenyl groups; phenyl groups which may optionally be substituted by a halogen atom or a $C_{1-6}$ alkyl or $C_{1-6}$ alkoxy group; phenyl-$C_{1-4}$alkyl groups; $C_{1-6}$ alkoxy groups which may optionally be substituted by a pyridyl, furyl or thienyl group; phenyl-$C_{1-4}$alkoxy groups which may optionally be substituted by a halogen atom or a $C_{1-6}$ alkyl, hydroxyl or $C_{1-6}$ alkoxy group; a phenoxy group; phenylaminocarbonyloxy groups which may optionally be substituted by a halogen atom; $C_{1-6}$ alkylaminocarbonyloxy groups; $C_{1-6}$ alkylthio groups; a phenylsulfonylamino group; amino groups which may optionally be substituted by a $C_{1-6}$ alkyl group; a hydroxyl group; a nitro group; an oxo group; phenyl-$C_{1-4}$alkylthio groups; phenyl-$C_{1-4}$alkylsulfonyl groups; a thienyl group; a pyridyl group; and lower alkylenedioxy groups; the substituent on $R^6$, when $R^6$ is a substituted amino group, is selected from the group consisting of $C_{1-6}$ alkyl groups which may optionally be substituted by a hydroxyl group; a $C_{1-6}$ acyl group; cycloalkyl groups; a pyridinecarbonyl group and an adamantyl group; and the substituent on $R^6$, when $R^6$ is a substituted nitrogen-containing heterocyclic group having a free valence on a nitrogen atom forming the heterocyclic ring, is selected from the group consisting of halogen atoms; a hydroxyl group; $C_{1-6}$ alkyl groups substituted by a hydroxyl group; an amino group; $C_{1-6}$ acyl groups which may optionally be substituted by a pyrrolidinyl group; phenyl-$C_{2-4}$alkenyl groups; aroyl groups which are substituted by a halogen atom; a pyridyl group and an oxo group.

**8.** Use of a 1,2-ethanediol derivative or a salt thereof according to Claim 7, wherein $R^1$ represents a phenyl group which may optionally be substituted by a halogen atom; a phenyl-$C_{1-4}$alkoxy group; a phenyl group; a thienyl group; a phenoxy group which may optionally be substituted by a halogen atom or a $C_{1-6}$ alkoxy group; or a $C_{1-6}$ alkoxy group which may optionally be substituted by a thienyl group; $R^2$ represents a hydrogen atom; $R^3$ represents a hydrogen atom; $nR^4$'s and $nR^5$'s represent hydrogen atoms; $R^6$ represents an imidazolyl group which may optionally be substituted by a $C_{1-6}$ alkyl group, or an amino group which may optionally be substituted by a $C_{1-6}$ alkyl or a cycloalkyl group.

**9.** Use of a 1,2-ethanediol derivative or a salt thereof according to Claim 5, wherein the derivative or salt is 1-(4-benzyloxyphenyl)-2-[2-(N,N-dimethylamino)-ethoxy]ethanol or a salt thereof.

**10.** Use of a 1,2-ethanediol derivative or a salt thereof according to Claim 5, wherein the derivative or salt is 2-[2-(N,N-dimethylamino)ethoxy]-1-(3-methyl-phenyl)ethanol or a salt thereof.

**11.** A cerebral function-improving agent comprising a 1,2-ethanediol derivative represented by the following general formula or a salt thereof:

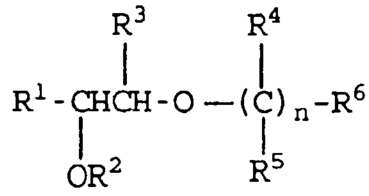

wherein $R^1$, $R^2$, $R^3$, $R^4$, $R^5$, $R^6$, n and all the heterocyclic groups are defined in Claim 5.

**Patentansprüche**

**1.** 1,2-Ethandiolderivat der folgenden allgemeinen Formel oder ein Salz davon:

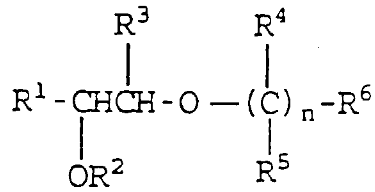

wobei $R^1$ für eine substituierte oder unsubstituierte Phenylgruppe steht; $R^2$ für ein Wasserstoffatom, eine $C_{1-6}$ Alkylgruppe oder eine Hydroxylschutzgruppe steht; $R^3$ für ein Wasserstoffatom oder eine $C_{1-6}$ Alkylgruppe steht, die $nR^4$ oder $nR^5$ jeweils gleich oder verschieden voneinander sein können und für Wasserstoffatome oder $C_{1-6}$ Alkylgruppen stehen; $R^6$ für substituiertes oder unsubstituiertes Amino oder eine Stickstoff enthaltende heterocyclische Gruppe steht, wobei die Stickstoff enthaltende heterocyclische Gruppe eine freie Valenz auf einem Stickstoffatom, das den heterocyclischen Ring bildet, aufweist und ausgewählt ist aus der Gruppe, bestehend aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen; und n für 0 oder eine ganze Zahl aus 1 bis 6 steht, wobei der Substituent von $R^1$ ausgewählt ist, aus der Gruppe bestehend aus Halogenatomen, substituierten oder unsubstituierten Amino-, $C_{1-6}$ Alkyl-, Aryl-, Ar-$C_{1-4}$-alkyl-, $C_{1-6}$ Alkoxy-, Ar-$C_{1-4}$-alkoxy-, Aryloxy-, Carbamoyloxy-, $C_{1-6}$ Alkylthio-, $C_{2-6}$ Alkenyl-, $C_{2-6}$ Alkenyloxy-, Ar-$C_{1-4}$-alkylthio-, Ar-$C_{1-6}$-alkylsulfonyl-, Arylsulfonyl-, $C_{1-6}$ Alkylsulfonylamino-, Arylsulfonylamino- und heterocyclischen Gruppen, geschützten Aminogruppen, geschützten oder ungeschützten Hydroxylgruppen, einer Nitrogruppe, einer Oxogruppe und $C_{1-4}$ Alkylendioxygruppen; die substituierte $C_{1-6}$ Alkyl-, Aryl-, Ar-$C_{1-4}$-alkyl-, $C_{1-6}$ Alkoxy-, Ar-$C_{1-4}$-alkoxy-, Aryloxy-, Carbamoyloxy-, $C_{1-6}$ Alkylthio-, $C_{2-6}$ Alkenyl-, $C_{2-6}$ Alkenyloxy-, Ar-$C_{1-4}$-alkylthio-, Ar-$C_{1-6}$-alkylsulfonyl-, Arylsulfonyl-, $C_{1-6}$ Alkylsulfonylamino-, Arylsulfonylamino- oder heterocyclische Gruppe als Substituent von $R^1$ und die substituierte, Stickstoff enthaltende, heterocyclische Gruppe von $R^6$ jeweils mindestens einen Substituenten aufweisen, der ausgewählt ist aus der Gruppe, bestehend aus Halogenatomen, geschützten Hydroxylgruppen, geschützten oder ungeschützten Aminogruppen, unsubstituierten $C_{1-6}$ Alkylgruppen, mit einer geschützten oder ungeschützten Hydroxylgruppe substituierten $C_{1-6}$ Alkylgruppen, mit Halogen substituierten Arylgruppen, mit Halogen substituierten Aroylgruppen, unsubstituierten $C_{1-6}$ Alkoxygruppen, mit einer $C_{1-6}$ Alkoxygruppe substituierten $C_{1-6}$ Alkoxygruppen, $C_{1-6}$ Acylgruppen, Ar-$C_{1-4}$-alkenylgruppen, heterocyclischen Gruppen, einer Oxogruppe, $C_{1-6}$ Alkylsulfonylgruppen und Arylsulfonylgruppen; und die substituierte Aminogruppe als Substituent von $R^1$ und die substituierte Aminogruppe als $R^6$ jeweils mindestens einen Substituenten aufweisen, der ausgewählt ist, aus der Gruppe, bestehend aus geschützten oder ungeschützten Hydroxylgruppen, unsubstituierten $C_{1-6}$ Alkylgruppen, mit einer geschützten oder ungeschützten Carboxyl- oder Hydroxylgruppe substituierten $C_{1-6}$ Alkylgruppen, Cycloalkylgruppen, Arylgruppen, $C_{1-6}$ Acylgruppen, unsubstituierten oder Oxo-substituierten heterocyclischen-CO-Gruppen, einer Adamantylgruppe, $C_{1-6}$ Alkylsulfonylgruppen und Arylsulfonylgruppen, mit der Maßgabe, daß diejenigen Verbindungen ausgeschlossen sind, bei denen $R^1$ für eine unsubstituierte oder mit $C_{1-6}$ Alkyl substituierte Phenylgruppe steht und $R^6$ für eine di-$C_{1-6}$ Alkylaminogruppe,

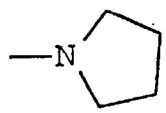, 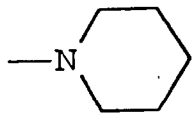 oder 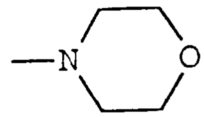

steht; wobei alle obigen heterocyclischen Gruppen ausgewählt sind aus der Gruppe, bestehend aus denjenigen Stickstoff enthaltenden heterocyclischen Gruppen, die bei der Definition von $R^6$ erwähnt wurden, sowie Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen.

**2.** 1,2-Ethandiolderivat oder ein Salz davon gemäß Anspruch 1, wobei $R^2$ für ein Wasserstoffatom oder eine Hydroxylschutzgruppe steht; die $nR^4$ gleich oder verschieden voneinander sind und für Wasserstoffatome oder $C_{1-6}$ Alkylgruppen stehen; die $nR^5$ für Wasserstoffatome stehen; und $R^6$ für eine substituierte oder unsubstituierte Aminogruppe (, die von einem Di-$C_{1-6}$-alkylamino verschieden ist) oder eine Stickstoff enthaltende heterocyclische Gruppe steht, wobei die Stickstoff enthaltende heterocyclische Gruppe eine freie Valenz auf einem Stickstoffatom, das den heterocyclischen Ring bildet, aufweist und ausgewählt ist aus der Gruppe, bestehend aus Pyrrolyl-, Piperazinyl-, Imidazolyl-, Pyrazolyl-, Pyridyl-, Pyrimidinyl-, Chinolyl-, Chinolizinyl-, Tetrahydrochinolinyl-, Chinuclidinyl-, Thiazolyl- und Thiadiazolylgruppen.

**3.** 1-(4-Benzyloxyphenyl)-2-[2-(N,N-dimethylamino)-ethoxy]ethanol oder ein Salz davon.

**4.** Verfahren zur Herstellung eines 1,2-Ethandiolderivats der folgenden allgemeinen Formel oder ein Salz davon:

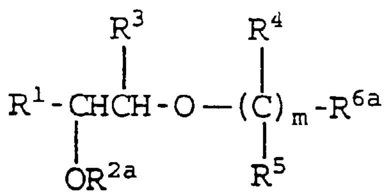

wobei $R^1$ für eine substituierte oder unsubstituierte Phenylgruppe steht; $R^{2a}$ für eine Hydroxylschutzgruppe steht; $R^3$, $R^4$, $R^5$ und alle heterocyclischen Gruppen wie in Anspruch 1 definiert sind und m dieselbe Bedeutung hat wie n, das in Anspruch 1 definiert wird und $R^{6a}$ dieselbe Bedeutung hat wie $R^6$, das in Anspruch 1 definiert wird, wobei das Verfahren umfaßt eine Umsetzung einer Verbindung der allgemeinen Formel:

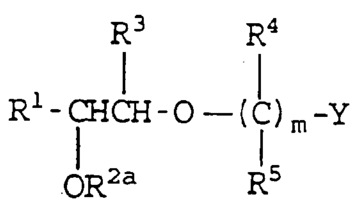

wobei $R^1$, $R^{2a}$, $R^3$, $R^4$, $R^5$ und m die oben definierten Bedeutungen haben und Y für eine abspaltbare Gruppe steht, mit einer Verbindung der folgenden allgemeinen Formel oder einem Salz davon:

$$H\text{-}R^{6a}$$

wobei $R^{6a}$ die oben definierte Bedeutung hat.

**5.** Verwendung eines 1,2-Ethandiolderivats der folgenden allgemeinen Formel oder eines Salzes davon zur Herstellung eines Medikaments zur Behandlung von zerebrovasculärer Demenz, Altersdemenz, Alzheimer-Demenz, Folgeerscheinungen von ischämischer Enzephalopathie oder zerebraler Apoplexie bei einem Patienten:

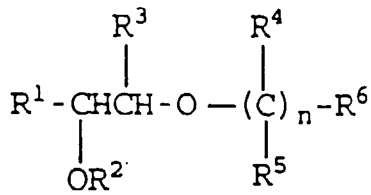

wobei $R^1$ für eine substituierte oder unsubstituierte Phenylgruppe steht; $R^2$ für ein Wasserstoffatom, eine $C_{1-6}$ Alkylgruppe oder eine Hydroxylschutzgruppe steht; $R^3$ für ein Wasserstoffatom oder ein $C_{1-6}$ Alkylgruppe steht; die $nR^4$ und $nR^5$ gleich oder verschieden voneinander sind und für Wasserstoffatome oder $C_{1-6}$ Alkylgruppen stehen; $R^6$ für eine Ammoniumgruppe oder eine substituierte oder unsubstituierte Aminogruppe oder eine Stickstoff enthaltende heterocyclische Gruppe steht, wobei die Stickstoff enthaltende heterocyclische Gruppe eine freie Valenz auf einem Stickstoffatom, das den heterocyclischen Ring bildet, aufweist und ausgewählt ist aus der Gruppe bestehend aus Pyrroloyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen; und n für 0 oder eine ganze Zahl von 1 bis 6 steht, wobei der Substituent von $R^1$ ausgewählt ist aus der Gruppe, bestehend aus Halogenatomen, substituierten oder unsubstituierten Amino-, $C_{1-6}$ Alkyl-, Aryl-, Ar-$C_{1-4}$-alkyl-, $C_{1-6}$ Alkoxy-, Ar-$C_{1-4}$-alkoxy-, Aryloxy-, Carbamoyloxy-, $C_{1-6}$ Alkylthio-, $C_{2-6}$ Alkenyl-, $C_{2-6}$ Alkenyloxy-, Ar-$C_{1-4}$-alkylthio-, Ar-$C_{1-6}$-alkylsulfonyl-, Arylsulfonyl-, $C_{1-6}$ Alkylsulfonylamino-, Arylsulfonylamino- und heterocyclischen Gruppen, geschützten Aminogruppen, geschützten oder ungeschützten Hydroxylgruppen, einer Nitrogruppe, einer Oxogruppe und $C_{1-4}$ Alkylendioxygruppen; die substituierte $C_{1-6}$ Alkyl-, Aryl-, Ar-$C_{1-4}$-alkyl-, $C_{1-6}$ Alkoxy-, Ar-$C_{1-4}$-alkoxy-, Aryloxy-, Carbamoyloxy-, $C_{1-6}$ Alkylthio-, $C_{2-6}$ Alkenyl-, $C_{2-6}$ Alkenyloxy-, Ar-$C_{1-4}$-alyklthio-, Ar-$C_{1-6}$-alkylsulfonyl-, Arylsulfonyl-, $C_{1-6}$ Alkylsulfonylamino-, Arylsulfonylamino- oder heterocyclische Gruppe als Substituent von $R^1$ und die substituierte Stickstoff enthaltende heterocyclische Gruppe als $R^6$ jeweils einen Substituenten aufweisen, der ausgewählt ist, aus der Gruppe, bestehend aus Halogenatomen, geschützten Hydroxylgruppen, geschützten oder ungeschützten Aminogruppen, ungeschützten $C_{1-6}$ Alkylgruppen, mit einer geschützten oder ungeschützten Hydroxylgruppe substituierten $C_{1-6}$ Alkylgruppen, mit Halogen substituierten Arylgruppen, mit Halogen substituierten Aroylgruppen, unsubstituierten $C_{1-6}$ Alkoxygruppen, mit einer $C_{1-6}$ Alkoxygruppe substituierten $C_{1-6}$ Alkoxygruppen, $C_{1-6}$ Acylgruppen, Ar-$C_{2-6}$-alkenylgruppen, heterocyclischen Gruppen, einer Oxogruppe, $C_{1-6}$ Alkylsulfonylgruppen und Arylsulfonylgruppen; und die substituierte Aminogruppe als Substituent von $R^1$ und die substituierte Aminogruppe als $R^6$ jeweils mindestens einen Substituenten aufweisen, der ausgewählt ist aus der Gruppe, bestehend aus geschützten oder ungeschützten Hydroxylgruppen, unsubstituierten $C_{1-6}$ Alkylgruppen, mit einer geschützten oder ungeschützten Carboxyl- oder Hydroxylgruppe substituierten $C_{1-6}$ Alkylgruppen, Cycloalkylgruppen, Arylgruppen, $C_{1-6}$ Acylgruppen, unsubstituierten oder Oxo-substituierten heterocyclischen-CO-Gruppen, Adamantylgruppen, $C_{1-6}$ Alkylsulfonylgruppen und Arylsulfonylgruppen; wobei alle obigen heterocyclischen Gruppen ausgewählt sind aus der Gruppe, bestehend aus den Stickstoff enthaltenden heterocyclischen Gruppen, die unter der Definition von $R_6$ erwähnt werden und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen.

**6.** Verwendung eines 1,2-Ethandiolderivats oder eines Salzes davon gemäß Anspruch 5, wobei $R^2$ für ein Wasserstoffatom oder eine Hydroxylschutzgruppe steht; die $nR^4$ gleich oder verschieden voneinander sind und für Wasserstoffatome oder $C_{1-6}$ Alkylgruppen stehen; die $nR^5$ für Wasserstoffatome stehen; und $R^6$ für eine Ammoniumgruppe oder eine substituierte oder unsubstituierte Aminogruppe oder eine Stickstoff enthaltende heterocyclische Gruppe steht, wobei die Stickstoff enthaltende heterocyclische Gruppe eine freie Valenz auf einem Stickstoffatom, das den heterocyclischen Ring bildet, aufweist und ausgewählt ist aus der Gruppe, bestehend aus Pyrrolyl-, Pyrrolidinyl-, Piperidinyl-, Piperazinyl-, Imidazolyl-, Pyrazolyl-, Pyridyl-, Pyrimidinyl-, Morpholinyl-, Chinolyl-, Chinolizinyl-, Tetrahydrochinolinyl-, Chinuclidinyl-, Thiazolyl- und Thiadiazolylgruppen.

7. Verwendung eines 1,2-Ethandiolderivats oder eines Salzes davon gemäß Anspruch 5, wobei $R^6$ für eine Ammoniumgruppe oder eine substituierte oder unsubstituierte Aminogruppe oder eine Stickstoff enthaltende heterocyclische Gruppe steht, wobei die Stickstoff enthaltende heterocyclische Gruppe eine freie Valenz auf einem Stickstoffatom, das den heterocyclischen Ring bildet, aufweist und ausgewählt ist aus der Gruppe, bestehend aus Pyrrolinyl-, Piperidyl-, Piperazinyl-, Imidazolyl-, Pyridyl-, Morpholinyl-, Thiomorpholinyl-, Tetrahydropyridyl-, Chinuclidinyl- und Tetrazolyl-, wobei der Substituent von $R^1$ ausgewählt ist aus der Gruppe, bestehend aus Halogenatomen; einer $C_{1-6}$ Alkylgruppe; $C_{2-6}$ Alkenylgruppen; Phenylgruppen, die gegebenenfalls mit einem Halogenatom oder einer $C_{1-6}$ Alkyl- oder $C_{1-6}$ Alkoxygruppe substituiert sein können; Phenyl-$C_{1-4}$alkylgruppen; $C_{1-6}$ Alkoxygruppen, die gegebenenfalls mit einer Pyridyl-, Furyl- oder Thienylgruppe substituiert sein können; Phenyl-$C_{1-4}$alkoxygruppen, die gegebenenfalls mit einem Halogenatom oder einer $C_{1-6}$ Alkyl-, Hydroxyl- oder $C_{1-6}$ Alkoxygruppe substituiert sein können; einer Phenoxygruppe; Phenylaminocarbonyloxygruppen, die gegebenenfalls mit einem Halogenatom substituiert sein können; $C_{1-6}$ Alkylaminocarbonyloxygruppen; $C_{1-6}$ Alkylthiogruppen; eine Phenylsulfonylaminogruppe; Aminogruppen, die gegebenenfalls mit einer $C_{1-6}$ Alkylgruppe substituiert sein können; eine Hydroxylgruppe; eine Nitrogruppe; eine Oxogruppe; Phenyl-$C_{1-4}$alkylthiogruppen; Phenyl-$C_{1-4}$alkylsulfonylgruppen; eine Thienylgruppe; eine Pyridylgruppe; und Niederalkylendioxygruppen; wobei der Substituent von $R^6$, für den Fall, daß $R^6$ für eine substituierte Aminogruppe steht, ausgewählt ist aus der Gruppe, bestehend aus $C_{1-6}$ Alkylgruppen, die gegebenenfalls mit einer Hydroxylgruppe substituiert sein können; einer $C_{1-6}$ Acylgruppe; Cycloalkylgruppen; einer Pyridincarbonylgruppe und einer Adamantylgruppe; und wobei der Substituent von $R^6$, für den Fall, daß $R^6$ für eine substituierte Stickstoff enthaltende heterocyclische Gruppe mit einer freien Valenz an einem Stickstoffatom, das den heterocyclischen Ring bildet, steht, ausgewählt ist aus der Gruppe, bestehend aus Halogenatomen; einer Hydroxylgruppe; mit einer Hydroxylgruppe substituierten $C_{1-6}$ Alkylgruppen; einer Aminogruppe; $C_{1-6}$ Acylgruppen, die gegebenenfalls mit einer Pyrrolidinylgruppe substituiert sein können; Phenyl-$C_{2-4}$alkenylgruppen; mit einem Halogenatom substituierten Aroylgruppen; einer Pyridylgruppe und einer Oxogruppe.

8. Verwendung eines 1,2-Ethandiolderivats oder eines Salzes davon gemäß Anspruch 7, wobei $R^1$ für eine gegebenenfalls mit einem Halogenatom substituierte Phenylgruppe; eine Phenyl-$C_{1-4}$alkoxygruppe; eine Phenylgruppe; eine Thienylgruppe; eine Phenoxygruppe, die gegebenenfalls mit einem Halogenatom oder einer $C_{1-6}$ Alkoxygruppe substituiert sein kann; oder eine $C_{1-6}$ Alkoxygruppe, die gegebenenfalls mit einer Thienylgruppe substituiert sein kann, steht; $R^2$ für ein Wasserstoffatom steht, $R^3$ für ein Wasserstoffatom steht; die $nR^4$ und $nR^5$ für Wasserstoffatome stehen; $R^6$ für eine gegebenenfalls mit einer $C_{1-6}$ Alkylgruppe substituierte Imidazolylgruppe oder eine gegebenenfalls mit einer $C_{1-6}$ Alkylgruppe oder einer Cycloalkylgruppe substituierte Aminogruppe steht.

9. Verwendung eines 1,2-Ethandiolderivats oder eines Salzes davon gemäß Anspruch 5, wobei das Derivat oder das Salz 1-(4-Benzyloxyphenyl)-2-[2-(N,N-dimethylamino)ethoxy]ethanol oder ein Salz davon ist.

10. Verwendung eines 1,2-Ethandiolderivats oder eines Salzes davon gemäß Anspruch 5, wobei das Derivat oder Salz 2-[2-(N,N-dimethylamino)ethoxy]-1-(3-methylphenyl)ethanol oder ein Salz davon ist.

11. Gehirnfunktionsverbesserndes Mittel, umfassend ein 1,2-Ethandiolderivat der folgenden allgemeinen Formel oder ein Salz davon:

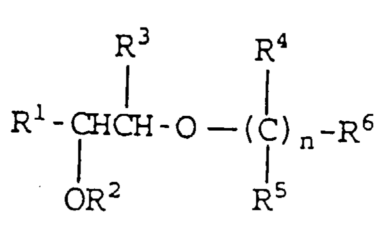

wobei $R^1$, $R^2$, $R^3$, $R^4$, $R^5$, $R^6$, n und alle heterocyclischen Gruppen wie in Anspruch 5 definiert sind.

**Revendications**

1. Dérivé d'éthanediol-1,2, représenté par la formule générale suivante, ou son sel :

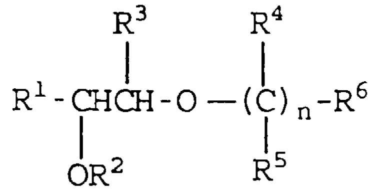

dans laquelle

$R^1$ représente un radical phényle substitué ou non substitué;
$R^2$ représente un atome d'hydrogène, un radical alcoyle en $C_{1-6}$ ou un groupe protecteur d'hydroxyle;
$R^3$ représente un atome d'hydrogène ou un radical alcoyle en $C_{1-6}$;
les n $R^4$ et les n $R^5$ sont identiques ou différents les uns des autres et représentent des atomes d'hydrogène ou des radicaux alcoyle en $C_{1-6}$;
$R^6$ représente un radical amino substitué ou non substitué ou hétérocyclique contenant un azote, substitué ou non substitué, le radical hétérocyclique contenant un azote ayant une valence libre sur un atome d'azote formant l'hétérocycle et étant choisi parmi le groupe constitué des radicaux pyrrolyle, pyrrolidinyle, pipéridinyle, pipérazinyle, imidazolyle, pyrazolyle, pyridyle, tétrahydropyridyle, pyrimidinyle, morpholinyle, thiomorpholinyle, quinoléyle, quinolizinyle, tétrahydroquinoléinyle, tétrahydroisoquinoléinyle, quinuclidinyle, thiazolyle, tétrazolyle, thiadiazolyle, pyrrolinyle, imidazolinyle, imidazolidinyle, pyrazolinyle, pyrazolidinyle, purinyle ou indazolyle, et
n représente 0 ou un entier de 1 à 6,

où le substituant sur $R^1$ est choisi parmi le groupe constitué des atomes d'halogène, amino substitué ou non substitué, alcoyle en $C_{1-6}$, aryle, ar(alcoyl en $C_{1-4}$), alcoxy en $C_{1-6}$, ar(alcoxy en $C_{1-4}$), aryloxy, carbamoyloxy, alcoylthio en $C_{1-6}$, alcényle en $C_{2-6}$, alcényloxy en $C_{2-6}$, ar(alcoyl en $C_{1-4}$)thio, ar(alcoyl en $C_{1-6}$)sulfonyle, arylsulfonyle, (alcoyl en $C_{1-6}$)sulfonylamino, arylsulfonylamino et radicaux hétérocycliques, radicaux amino protégés, radicaux hydroxyle protégés et non protégés, radical nitro, radical oxo et radicaux alcoylènedioxy en $C_{1-4}$; les radicaux substitués alcoyle en $C_{1-6}$, aryle, ar(alcoyl en $C_{1-4}$), alcoxy en $C_{1-6}$, ar(alcoxy en $C_{1-4}$), aryloxy, carbamoyloxy, alcoylthio en $C_{1-6}$, alcényle en $C_{2-6}$, alcényloxy en $C_{2-6}$, ar(alcoyl en $C_{1-4}$)thio, ar(alcoyl en $C_{1-6}$)sulfonyle, arylsulfonyle, (alcoyl en $C_{1-6}$)sulfonylamino, arylsulfonylamino ou hétérocycliques comme substituant de $R^1$ et le radical hétérocyclique contenant un azote, substitué, en tant que $R^6$ ont chacun au moins un substituant choisi dans le groupe constitué des atomes d'halogène, radicaux hydroxyle protégés, radicaux amino protégés ou non protégés, radicaux alcoyle en $C_{1-6}$ non substitués, radicaux alcoyle en $C_{1-6}$ substitués par un radical hydroxyle protégé ou non protégé, radicaux aryle halogénosubstitués, radicaux aroyle halogénosubstitués, radicaux alcoxy en $C_{1-6}$ non substitués, radicaux alcoxy en $C_{1-6}$ substitués par un radical alcoxy en $C_{1-6}$, radicaux acyle en $C_{1-6}$, radicaux ar(alcényl en $C_{1-4}$), radicaux hétérocycliques, radical oxo, radicaux alcoylsulfonyle en $C_{1-6}$ et radicaux arylsulfonyle; et le radical amino substitué comme substituant de $R^1$ et le radical amino substitué comme $R^6$ ont chacun au moins un substituant choisi parmi le groupe constitué des radicaux hydroxyle protégés ou non protégés, radicaux alcoyle en $C_{1-6}$ non substitués, radicaux alcoyle en $C_{1-6}$ substitués par un radical carboxyle ou hydroxyle protégé ou non protégé, radicaux cycloalcoyle, radicaux aryle, radicaux acyle en $C_{1-6}$, radicaux -CO-hétérocycliques non susbtitués ou oxosubstitués, radical adamantyle, radicaux alcoylsulfonyle en $C_{1-6}$ et radicaux arylsulfonyle, pourvu que l'on exclue les composés dans lesquels $R^1$ représente un radical phényle non substitué ou substitué par un alcoyle en $C_{1-6}$ et $R^6$ représente un radical di(alcoyl en $C_{1-6}$) amino,

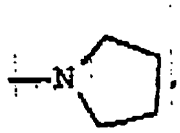, 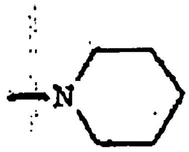 ou 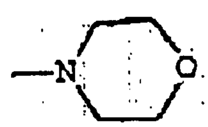

tous les radicaux hétérocycliques ci-dessus étant choisis parmi le groupe constitué des radicaux hétérocycliques contenant un azote, mentionnés dans la définition de $R^6$ et les radicaux furyle, thiényle, benzothiényle, pyrannyle, isobenzofurannyle, oxazolyle, benzofurannyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofurannyle, benzo[b]dioxannyle, imidazo[2,3-a]pyridyle, benzo[b]pipérazinyle, chroményle, isothiazolyle, isoxazolyle, oxadiazolyle, pyridazinyle, isoindolyle et isoquinoléyle.

**2.** Dérivé d'éthanediol-1,2 ou son sel suivant la revendication 1, dans lequel :

$R^2$ représente un atome d'hydrogène ou un groupe protecteur d'hydroxyle;
les n $R^4$ sont identiques ou différents les uns des autres et représentent des atomes d'hydrogène ou des radicaux alcoyle en $C_{1-6}$;
les n $R^5$ représentent des atomes d'hydrogène, et
$R^6$ représente un radical amino substitué ou non substitué [autre que di(alcoyl en $C_{1-6}$)amino] ou hétérocyclique contenant un azote substitué ou non substitué, le radical hétérocyclique contenant un azote ayant une valence libre sur un atome d'azote formant l'hétérocycle et étant choisi parmi le groupe constitué des radicaux pyrrolyle, pipérazinyle, imidazolyle, pyrazolyle, pyridyle, pyrimidinyle, quinoléyle, quinolizinyle, tétrahydroquinoléinyle, quinuclidinyle, thiazolyle et thiadiazolyle.

**3.** Le 1-(4-benzyloxyphényl)-2-[2-(N,N-diméthylamino)éthoxy]éthanol ou son sel.

**4.** Procédé de préparation d'un dérivé d'éthanediol-1,2 représenté par la formule générale suivante, ou son sel :

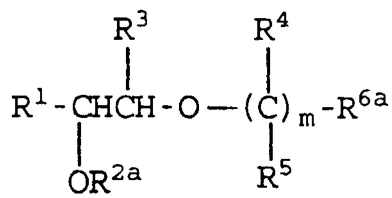

dans laquelle :

$R^1$ représente un radical phényle substitué ou non substitué;
$R^{2a}$ représente un groupe protecteur d'hydroxyle;
$R^3$, $R^4$, $R^5$ et tous les radicaux hétérocycliques sont tels que définis à la revendication 1, et
m est identique à n défini à la revendication 1, et
$R^{6a}$ est identique à $R^6$ défini à la revendication 1, qui comprend la réaction d'un composé représenté par la formule générale :

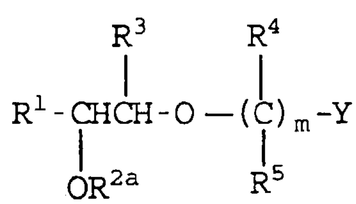

dans laquelle :
$R^1$ , $R^{2a}$, $R^3$, $R^4$, $R^5$ et m ont les mêmes significations que défini ci-dessus et Y représente un groupe éliminable,

avec un composé représenté par la formule générale suivante ou son sel :

$$H\text{-}R^{6a}$$

dans laquelle :

$R^{6a}$ a la même signification que défini ci-dessus.

**5.** Utilisation d'un dérivé d'éthanediol-1,2 représenté par la formule générale suivante ou son sel, dans la préparation d'un médicament pour traiter la démence cérébrovasculaire, la démence sénile, la maladie d'Alzheimer, les séquelles de l'encéphalopathie ischémique ou de l'apoplexie cérébrale chez un patient :

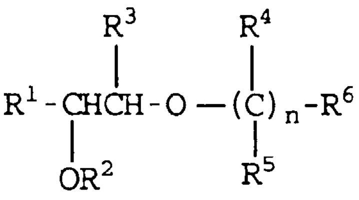

dans laquelle :

$R^1$ représente un radical phényle substitué ou non substitué;
$R^2$ représente un atome d'hydrogène, un radical alcoyle en $C_{1-6}$ ou un groupe protecteur d'hydroxyle;
$R^3$ représente un atome d'hydrogène ou un radical alcoyle en $C_{1-6}$;
les n $R^4$ et les n $R^5$ sont identiques ou différents les uns des autres et représentent des atomes d'hydrogène ou des radicaux alcoyle en $C_{1-6}$;
$R^6$ représente un radical ammonio ou radical amino substitué ou non substitué ou hétérocyclique contenant un azote, substitué ou non substitué, le radical hétérocyclique contenant un azote ayant une valence libre sur un atome d'azote formant l'hétérocycle et étant choisi parmi le groupe constitué des radicaux pyrrolyle, pyrrolidinyle, pipéridinyle, pipérazinyle, imidazolyle, pyrazolyle, pyridyle, tétrahydropyridyle, pyrimidinyle, morpholinyle, thiomorpholinyle, quinoléyle, quinolizinyle, tétrahydroquinoléinyle, tétrahydroisoquinoléinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle, et
n représente 0 ou un entier de 1 à 6,

où le substituant sur $R^1$ est choisi parmi le groupe constitué des atomes d'halogène, amino substitué ou non substitué, alcoyle en $C_{1-6}$, aryle, ar(alcoyl en $C_{1-4}$), alcoxy en $C_{1-6}$, ar(alcoxy en $C_{1-4}$), aryloxy, carbamoyloxy, alcoylthio en $C_{1-6}$, alcényle en $C_{2-6}$, alcényloxy en $C_{2-6}$, ar(alcoyl en $C_{1-4}$)thio, ar(alcoyl en $C_{1-6}$)sulfonyle, arylsulfonyle, (alcoyle en $C_{1-6}$)sulfonylamino, arylsulfonylamino et des radicaux hétérocycliques, radicaux amino protégés, radicaux hydroxyle protégés et non protégés, radical nitro, radical oxo et radicaux alcoylènedioxy en $C_{1-4}$; les radicaux substitués alcoyle en $C_{1-6}$, aryle, ar(alcoyl en $C_{1-4)}$, alcoxy en $C_{1-6}$, ar(alcoxy en $C_{1-4}$), aryloxy, carbamoyloxy, alcoylthio en $C_{1-6}$, alcényle en $C_{2-6}$, alcényloxy en $C_{2-6}$, ar(alcoyl en $C_{1-4}$)thio, ar(alcoyl en $C_{1-6}$)sulfonyle, arylsulfonyle, (alcoyl en $C_{1-6}$)sulfonylamino, arylsulfonylamino ou hétérocycliques comme substituant de $R^1$ et le radical hétérocyclique contenant un azote, substitué, en tant que $R^6$ ont chacun au moins un susbtituant choisi parmi le groupe constitué des atomes d'halogène, radicaux hydroxyle protégés, radicaux amino protégés ou non protégés, radicaux alcoyle en $C_{1-6}$ non substitués, radicaux alcoyle en $C_{1-6}$ substitués par un radical hydroxyle protégé ou non protégé, radicaux aryle halogénosubstitués, radicaux aroyle halogénosubstitués, radicaux alcoxy en $C_{1-6}$ non substitués, radicaux alcoxy en $C_{1-6}$ substitués par un radical alcoxy en $C_{1-6}$, radicaux acyle en $C_{1-6}$, radicaux ar(alcényl en $C_{2-6}$), radicaux hétérocycliques, radical oxo, radicaux alcoylsufonyle en $C_{1-6}$ et radicaux arylsulfonyle; et le radical amino substitué comme substituant de $R^1$ et le radical amino substitué comme $R^6$ ont chacun au moins un substituant choisi parmi le groupe constitué des radicaux hydroxyle protégés ou non protégés, radicaux alcoyle en $C_{1-6}$ non substitués, radicaux alcoyle en $C_{1-6}$ substitués par un radical carboxyle ou hydroxyle protégé ou non protégé, radicaux cycloalcoyle, radicaux aryle, radicaux acyle en $C_{1-6}$, radicaux -CO-hétérocycliques non substitués ou oxosubstitués, radical adamantyle, radicaux alcoylsulfonyle en $C_{1-6}$ et radicaux arylsulfonyle; tous les radicaux hétérocycliques ci-dessus étant choisis parmi le groupe constitué des radicaux hétérocycliques contenant un azote, mentionnés dans la définition de $R^6$ et les radicaux furyle, thiényle, benzothiényle, pyrannyle, isobenzofurannyle, oxazolyle, benzofurannyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofurannyle, benzo[b]dioxannyle, imidazo[2,3-a]pyridyle, benzo[b]pipérazinyle, chroményle, isothiazolyle, isoxazolyle, oxadiazolyle, pyridazinyle, isoindolyle et isoquinoléyle.

6. Utilisation d'un dérivé d'éthanediol-1,2 ou de son sel suivant la revendication 5, dans laquelle :

$R^2$ représente un atome d'hydrogène ou un groupe protecteur d'hydroxyle;
les n $R^4$ sont identiques ou différents les uns des autres et représentent des atomes d'hydrogène ou des radicaux alcoyle en $C_{1-6}$;
les n $R^5$ représentent des atomes d'hydrogène, et
$R^6$ représente un radical ammonio ou un radical amino substitué ou non substitué ou hétérocyclique contenant un azote, substitué ou non substitué, le radical hétérocyclique contenant un azote ayant une valence libre sur un atome d'azote formant l'hétérocycle et étant choisi parmi le groupe constitué des radicaux pyrrolyle, pyrrolidinyle, pipéridinyle, pipérazinyle, imidazolyle, pyrazolyle, pyridyle, pyrimidinyle, morpholinyle, quinoléyle, quinolizinyle, tétrahydroquinoléinyle, quinuclidinyle, thiazolyle et thiadiazolyle.

7. Utilisation d'un dérivé d'éthanediol-1,2 ou de son sel suivant la revendication 5, dans laquelle :
$R^6$ représente un radical ammonio ou un radical amino substitué ou non substitué ou hétérocyclique contenant un azote, substitué ou non substitué, le radical hétérocyclique contenant un azote ayant une valence libre sur un atome d'azote formant l'hétérocycle et étant choisi parmi le groupe constitué des radicaux pyrolinyle, pipéridyle, pipérazinyle, imidazolyle, pyridyle, morpholinyle, thiomorpholinyle, tétrahydropyridyle, morpholinyle, thiomorpholinyle, tétrahydropyridyle, quinuclidinyle et tétrazolyle, le substituant sur $R^1$ est choisi parmi le groupe constitué des atomes d'halogène; radical alcoyle en $C_{1-6}$; radicaux alcényle en $C_{2-6}$; radicaux phényle qui peuvent être facultativement substitués par un atome d'halogène ou un radical alcoyle en $C_{1-6}$ ou alcoxy en $C_{1-6}$; radicaux phényl(alcoyle en $C_{1-4}$) ; radicaux alcoxy en $C_{1-6}$ qui peuvent être facultativement substitués par un radical pyridyle, furyle ou thiényle; radicaux phényl(alcoxy en $C_{1-4}$) qui peuvent être facultativement substitués, par un atome d'halogène ou un radical alcoyle en $C_{1-6}$, hydroxyle ou alcoxy en $C_{1-6}$; radical phénoxy; radicaux phénylaminocarbonyloxy qui peuvent être facultativement substitués par un atome d'halogène; radicaux (alcoyl en $C_{1-6}$)aminocarbonyloxy; radicaux alcoylthio en $C_{1-6}$; radical phénylsulfonylamino; radicaux amino qui peuvent être facultativement substitués par un radical alcoyle en $C_{1-4}$ ; radical hydroxyle; radical nitro; radical oxo; radicaux phényl (alcoyl en $C_{1-4}$)thio; radicaux phényl(alcoyl en $C_{1-4}$)sulfonyle; radical thiényle; radical pyridyle; et les radicaux alcoylènedioxy inférieurs; le substituant sur $R^6$, lorsque $R^6$ est un radical amino substitué, est choisi parmi le groupe constitué des radicaux alcoyle en $C_{1-6}$ qui peuvent être facultativement substitués par un radical hydroxyle; radical acyle en $C_{1-6}$; radicaux cycloalcoyle, radical pyridinecarbonyle et radical adamantyle; et le substituant sur $R^6$ lorsque $R^6$ est un radical hétérocyclique contenant un azote, substitué, ayant une valence libre sur un atome d'azote formant l'hétérocycle, est choisi parmi le groupe constitué des atomes d'halogène; radical hydroxyle; radicaux alcoyle en $C_{1-6}$ substitués par un radical hydroxyle; radical amino; radicaux acyle en $C_{1-6}$ qui peuvent être facultativement substitués par un radical pyrrolidinyle; radicaux phényl(alcényl en $C_{2-6}$); radicaux aroyle qui sont substitués par un atome d'halogène; radical pyridyle et radical oxo.

8. Utilisation d'un dérivé d'éthanediol-1,2 ou de son sel suivant la revendication 7, dans laquelle :

$R^1$ représente un radical phényle qui, peut être facultativement substitué par un atome d'halogène; un radical phényl(alcoxy en $C_{1-4}$); un radical phényle; un radical thiényle; un radical phénoxy qui peut être facultativement substitué par un atome d'halogène ou un radical alcoxy en $C_{1-6}$; ou un radical alcoxy en $C_{1-6}$ qui peut être facultativement substitué par un radical thiényle;
$R^2$ représente un atome d'hydrogène;
$R^3$ représente un atome d'hydrogène;
les n $R^4$ et les n $R^5$ représentent des atomes d'hydrogène;
$R^6$ représente un radical imidazolyle qui peut être facultativement substitué par un radical alcoyle en $C_{1-6}$, ou un radical amino qui peut être facultativement substitué par un radical alcoyle en $C_{1-6}$ ou un radical cycloalcoyle.

9. Utilisation d'un dérivé d'éthanediol-1-2 ou de son sel suivant la revendication 5, dans laquelle le dérivé ou le sel est le 1-(4-benzyloxyphényl)-2-[2-(N,N-diméthylamino)éthoxy]éthanol ou son sel.

10. Utilisation d'un dérivé d'éthanediol-1,2 ou de son sel suivant la revendication 5, dans laquelle le dérivé ou le sel est le 2-[2-(N,N-diméthylamino)éthoxy]-1-(3-méthylphényl)éthanol ou son sel.

11. Agent rétablissant la fonction cérébrale comprenant un dérivé d'éthanediol-1,2 représenté par la formule générale suivante, ou son sel :

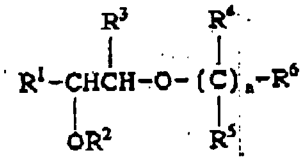

dans laquelle :

$R^1$, $R^2$, $R^3$, $R^4$, $R^5$, $R^6$, n et tous les radicaux hétérocycliques sont définis à la revendication 5.